(19) Europäisches Patentamt
European Patent Office
Office européen des brevets

(11) Veröffentlichungsnummer: **0 314 615 B1**

(12) **EUROPÄISCHE PATENTSCHRIFT**

(45) Veröffentlichungstag der Patentschrift: **26.04.95**

(51) Int. Cl.$^{6}$: **C07D 401/12**, C07D 253/06, C07D 271/10, A01N 43/707

(21) Anmeldenummer: **88810692.9**

(22) Anmeldetag: **07.10.88**

# (54) Schädlingsbekämpfungsmittel.

(30) Priorität: **16.10.87 CH 4062/87**

(43) Veröffentlichungstag der Anmeldung:
**03.05.89 Patentblatt 89/18**

(45) Bekanntmachung des Hinweises auf die Patenterteilung:
**26.04.95 Patentblatt 95/17**

(84) Benannte Vertragsstaaten:
**AT BE CH DE ES FR GB IT LI NL**

(56) Entgegenhaltungen:
**DE-A- 1 795 785**

**J. LIEBIGS : "ANNALEN DER CHEMIE, Band 749, 1971, Verlag Chemie GmbH, Weinheim, DE Seiten 125-133: A. HETZHEIM et al.: "Ringumwandlung von 2-Amino-3-phenacyl-1.3.4-oxadiazoliumhalogeniden mit Hydrazinen in as-Triazine"**

(73) Patentinhaber: **CIBA-GEIGY AG**
**Klybeckstrasse 141**
**CH-4002 Basel (CH)**

(72) Erfinder: **Kristinsson, Haukur, Dr.**
**Leimenstrasse 30**
**CH-4051 Basel (CH)**

EP 0 314 615 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Vorliegende Erfindung betrifft neue insektizid wirksame N-Amino-1,2,4-triazinone, Verfahren und Zwischenprodukte zu ihrer Herstellung, Mittel, welche diese Aminotriazinone enthalten und ihre Verwendung in der Schädlingsbekämpfung.

Die erfindungsgemässen Aminotriazinone entsprechen der Formel I

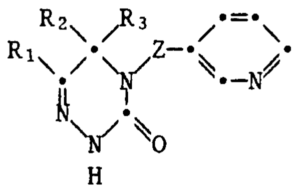

(I),

worin

$R_1$ Wasserstoff, $C_1$-$C_{12}$-Alkyl, $C_3$-$C_6$-Cycloalkyl, $C_1$-$C_4$-Alkoxy-$C_1$-$C_6$-alkyl, $C_1$-$C_2$-Halogenalkyl, Phenyl, Benzyl, Phenäthyl, Phenpropyl, Phenbutyl, Phenpentyl oder einen ein- oder zweifach durch Halogen, $C_1$-$C_5$-Alkyl, $C_1$-$C_2$-Halogenalkyl, Methoxy und/oder Aethoxy substituierten Phenyl-, Benzyl-, Phenäthyl-, Phenpropyl-, Phenbutyl- oder Phenpentylrest,

$R_2$ Wasserstoff, $C_1$-$C_6$-Alkyl, $C_3$-$C_6$-Cycloalkyl, unsubstituiertes oder durch $C_1$-$C_{12}$-Alkyl, Halogen oder $C_1$-$C_{12}$-Halogenalkyl substituiertes Phenyl oder $R_1$ und $R_2$ zusammen einen gesättigten oder ungesättigten 3- bis 7-gliedrigen Carbocyclus bilden,

$R_3$ Wasserstoff oder $C_1$-$C_6$-Alkyl bedeuten und

Z für -N=CH- oder -NH-$CH_2$- steht,

Die Verbindungen der Formel I können auch in Form von Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, Salpetersäure, verschiedene Phosphorsäuren, Schwefelsäure, Essigsäure, Propionsäure, Buttersäure, Valeriansäure, Oxalsäure, Malonsäure, Maleinsäure, Fumarsäsure, Milchsäure, Weinsäure oder Salicylsäure.

Die als Substituenten in Betracht kommenden Alkyle können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl, Decyl, Dodecyl usw. und ihre Isomeren genannt.

Die als Substituenten in Betracht kommenden Alkoxyalkyle können geradkettig oder verzweigt sein, wobei für die Alkyl- und Alkoxyreste die oben gegebenen Definitionen gelten. Geeignete Beispiele solcher Substituenten sind u.a. Methoxymethyl, Methoxyäthyl, Aethoxyäthyl, Methoxypropyl, Aethoxypropyl, Propoxypropyl, Methoxybutyl, Aethoxybutyl, Propoxybutyl oder Butoxybutyl.

Bei den als Substituenten in Betracht kommenden Cycloalkylen handelt es sich beispielsweise um Cyclopropyl, Cyclobutyl, Cyclopentyl oder Cyclohexyl.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden halogenierten $C_1$-$C_2$-Alkyle können nur teilweise oder auch perhalogeniert sein, wobei für die Halogene die oben gegebene Definition gilt. Besonders geeignete Beispiele solcher Substituenten sind u.a. das ein- bis dreifach durch Fluor, Chlor und/oder Brom substituierte Methyl wie z.B. $CHF_2$ oder $CF_3$; und das ein-bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CHBr_2$, $CF_2CHClF$, $CF_2CHBrF$ oder $CClFCHClF$.

Die durch $R_1$ und $R_2$ gebildeten 3- bis 7-gliedrigen Carbocyclen können gesättigt oder ungesättigt sein. Vorzugsweise handelt es sich um gesättigte 5- oder 6-gliedrige Carbocyclen.

Unter den Verbindungen der Formel I stehen diejenigen im Vordergrund, in denen $R_1$ Wasserstoff, $C_1$-$C_6$-Alkyl, $C_3$-$C_5$-Cycloalkyl, Phenyl oder ein-oder zweifach durch Halogen $C_1$-$C_3$-Alkyl, Methoxy oder Aethoxy substituiertes Phenyl; $R_2$ und $R_3$ je Wasserstoff oder $C_1$-$C_4$-Alkyl bedeuten und Z für -N=CH- oder -NH-$CH_2$-steht.

Dabei sind diejenigen Verbindungen der Formel I bevorzugt, in denen

a) $R_1$ für Wasserstoff, $C_1$-$C_4$-Alkyl, Cyclopropyl oder Phenyl; $R_2$ für Wasserstoff, Methyl oder Aethyl; $R_3$ für Wasserstoff oder Methyl; und Z für -N=CH-stehen oder

b) $R_1$ für Wasserstoff, $C_1$-$C_4$-Alkyl, Cyclopropyl oder Phenyl; $R_2$ für Wasserstoff, Methyl oder Aethyl; $R_3$ für Wasserstoff oder Methyl; und Z für -NH-$CH_2$-stehen.

Die erfindungsgemässen Verbindungen der Formel I können nach im Prinzip bekannten Verfahren hergestellt werden, indem man z.B.

A) ein Aminotriazinon der Formel II

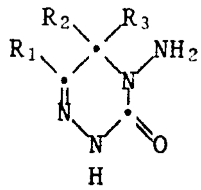

(II)

mit einem Aldehyd der Formel III

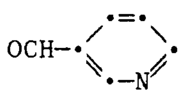

(III)

umsetzt und gegebenenfalls

B) das entstandene Pyridyl-methylenamino-triazinon der Formel Ia

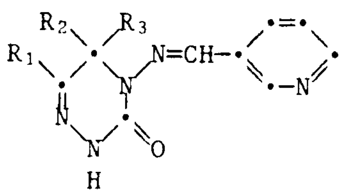

(Ia)

durch selektive Reduktion in das Pyridyl-methylamino-triazinon der Formel Ib

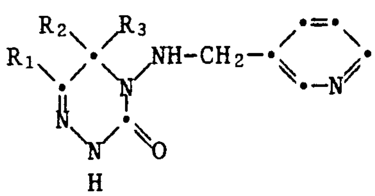

(Ib)

überführt. Dabei haben $R_1$, $R_2$ und $R_3$ die für die Formel I angegebene Bedeutung.

Das Verfahren A wird üblicherweise unter normalem Druck in Gegenwart einer katalytischen Menge einer starken Säure und in einem Lösungsmittel durchgeführt. Die Reaktionstemperatur beträgt dabei zwischen +10 und 100°C, vorzugsweise +40 bis 80°C. Als Säuren eignen sich starke anorganische Säuren wie z.B. Mineralsäuren, insbesondere Salzsäure. Als Lösungsmittel eigenen sich Alkohole, Aether und ätherartige Verbindungen, Nitrile oder auch Wasser.

Das Verfahren B wird üblicherweise unter normalem oder leicht erhöhtem Druck in Gegenwart eines geeigneten Hydrierungskatalysators und in einem Lösungsmittel durchgeführt. Geeignete Hydrierungskatalysatoren sind die üblichen Platin-, Palladium- oder Nickel-Katalysatoren wie z.B. Raney-Nickel oder auch Hydride wie z.B. Natrium-Borhydrid. Als Lösungsmittel eigenen sich Alkohole, Essigsäure, Essigester oder auch Wasser.

Die Aminotriazinone der Formel II können z.B. durch eine Ringumlagerung mit Hydrazinhydrat hergestellt werden, indem man ein Oxadiazolon der Formel IV

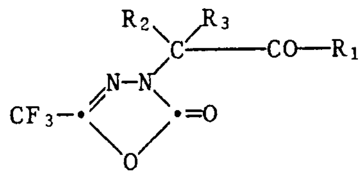

(IV)

mit Hydrazinhydrat ($H_2N\text{-}NH_2 \cdot H_2O$) umsetzt, wobei $R_1$, $R_2$ und $R_3$ die für Formel I angegebene Bedeutung haben.

Das Verfahren zur Herstellung der Aminotriazinone der Formel II wird üblicherweise unter normalem Druck und gegebenenfalls in einem Lösungsmittel durchgeführt. Die Temperatur beträgt dabei zwischen +15 und 120 °C, vorzugsweise zwischen +20 und 80 °C. Geeignete Lösungsmittel sind u.a. Wasser, Nitrile wie Acetonitril, Alkohole, Dioxan oder Tetrahydrofuran.

Die Oxadiazolone der Formel IV können nach im Prinzip bekannten Verfahren hergestellt werden, indem man z.B. das 5-Trifluormethyl-1,3,4-oxadiazol-2(3H)-on der Formel V

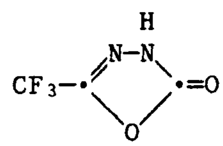

(V)

mit einem Keton der Formel VI

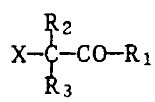

(VI)

umsetzt wobei $R_1$, $R_2$ und $R_3$ die für Formel I angegebene Bedeutung haben und X für Halogen steht.

Das Verfahren zur Herstellung der Oxadiazolone der Formel IV wird bei normalem Druck in Gegenwart einer Base und in einem Lösungsmittel durchgeführt. Die Temperatur beträgt dabei zwischen 0 und +150 °C, vorzugsweise zwischen +20 und 100 °C. Als Basen eignen sich organische und anorganische Basen wie z.B. Trimethylamin, Alkoholate, Natriumhydroxid oder Natriumhydrid. Als Lösungsmittel eigenen sich u.a. Alkohole, halogenierte Kohlenwasserstoffe wie z.B. Chloroform, Nitrile wie z.B. Acetonitril, Tetrahydrofuran, Dioxan, Dimethylsulfoxid oder auch Wasser.

Von den Aminotriazinonen der Formel II ist das 4-Amino-6-phenyl-1,2,4-triazin-3-on bekannt (Liebigs Annalen der Chemie, 749, 125 (1971), d.h. die Verbindung der Formel II, in der $R_1$ Phenyl, und $R_2$ und $R_3$ je Wasserstoff sind. Alle übrigen Verbindungen der Formel II, d.h. also die Verbindungen der Formel IIa

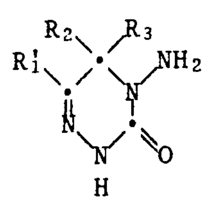

(IIa),

worin

$R_1'$ Wasserstoff, $C_1$-$C_{12}$-Alkyl, $C_3$-$C_6$-Cycloalkyl, $C_1$-$C_4$-Alkoxy-$C_1$-$C_6$-alkyl, $C_1$-$C_2$-Halogenalkyl, Benzyl, Phenäthyl, Phenpropyl, Phenbutyl, Phenpentyl oder einen ein- oder zweifach durch Halogen, $C_1$-$C_5$-Alkyl, $C_1$-$C_2$-Halogenalkyl, Methoxy und/oder Aethoxy substituierten Phenyl-, Benzyl-, Phenäthyl-, Phenpropyl-, Phenbutyl- oder Phenpentylrest,

$R_2$ Wasserstoff, $C_1$-$C_6$-Alkyl, $C_3$-$C_6$-Cycloalkyl, unsubstituiertes oder durch $C_1$-$C_{12}$-Alkyl, Halogen oder $C_1$-$C_{12}$-Halogenalkyl substituiertes Phenyl oder $R_1$ und $R_2$ zusammen einen gesättigten

oder ungesättigten 3- bis 7-gliedrigen Carbocyclus bilden,

$R_3$ Wasserstoff oder $C_1$-$C_6$-Alkyl bedeuten,

und die Oxadiazolone der Formel IV sind neu und bilden ebenfalls einen Gegenstand der Erfindung.

Die Verbindungen der Formeln III, V und VI sind bekannt oder können nach im Prinzip bekannten Verfahren hergestellt werden.

Es wurde nun gefunden, dass die erfindungsgemässen Verbindungen der Formel I bei guter Pflanzenverträglichkeit eine bessere Warmblüterverträglichkeit und eine höhere Stabilität aufweisen als bekannte Phosphorsäureester und Carbamate. Sie eignen sich deshalb ausgezeichnet als Schädlingsbekämpfungsmittel, vor allem zur Bekämpfung von Pflanzen und Tiere befallenden Schädlingen, insbesondere von Insekten.

Die Verbindungen der Formel I eignen sich insbesondere zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Hierzu werden die Schädlinge in ihren verschiedenen Entwicklungsstadien mit einer Verbindung der Formel I oder einem Salz davon gemäß Anspruch 1 in kontakt gebracht.

Mit Hilfe der erfindungsgemäss verwendeten Verbindungen der Formel I können vor allem pflanzenschädigende Insekten, speziell pflanzenschäigende Insekten in Zier- und Nutzpflanzungen, insbesondere in Baumwollkulturen, Gemüsekulturen, Reiskulturen und Obstkulturen bekämpft werden. In diesem Zusammenhang ist hervorzuheben, dass die genannten Verbindungen sich sowohl durch eine stark ausgeprägte systemische als auch Kontakt-Wirkung gegen saugende Insekten, vor allem gegen Insekten der Familie Aphididae (wie z.B. Aphis fabae, Aphis craccivora und Myzus persicae), welche sich mit herkömmlichen Mitteln nur schwierig bekämpfen lassen, auszeichnen.

Die gute pestizide Wirkung der erfindungsgemäss vorgeschlagenen Verbindungen der Formel I entspricht einer Abtötungsrate (Mortalität) von mindestens 50-60 % der erwähnten Schädlinge.

Die Wirkung der erfindungsgemäss verwendeten Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden als Schädlingsbekämpfungsmittel in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{12}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäureoder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"MC Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien 1981.

Die erfindungsgemässen pestiziden Zubereitungen enthalten in der Regel 0,1 bis 99 %, insbesondere 0,1 bis 95 %, eines Wirkstoffes der Formel I oder Kombinationen davon mit andern Insektiziden oder Akariziden, 1 bis 99,9 % eines festen oder flüssigen Zusatzstoffes und 0 bis 25 %, insbesondere 0,1 bis 20 %, eines Tensides. Während
als Handelsware eher konzentrierte Mittel bevorzugt werden, verwendet der Endverbraucher in der Regel verdünnte Zubereitungen, die wesentlich geringere Wirkstoffkonzentrationen aufweisen.

Die erfindungsgemässen Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiele:

1. Herstellung der Verbindungen der Formel I und ihrer Zwischenprodukte.

Beispiel H.1: 2-Oxo-5-trifluormethyl-2,3-dihydro-1,3,4-oxadiazol-3-aceton.

15 g (0,5 Mol) von 80%-iger NaH-Dispersion in Oel werden mit Petroläther ölfrei gewaschen und mit 125 ml DMF vorgelegt. Zu dieser Suspension werden 77 g (0.5 Mol) 5-Trifluormethyl-1,3,4-oxadiazol-2(3H)-on in 250 ml DMF bei Raumtemperatur innert 1 Stunde zugetropft und anschliessend für 3 Stunden gerührt. Anschliessend werden 55,5 g (0,6 Mol) Chloraceton zugegeben und das Reaktionsgemisch während 16 Stunden bei Raumtemperatur gerührt. Nach dem Eindampfen wird der Rückstand mit 1000 ml Wasser versetzt und der feste Niederschlag abgesaugt und getrocknet. Die Titelverbindung der Formel

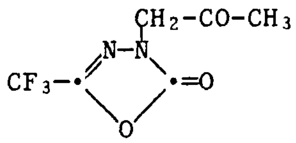

(Verbindung Nr. 1.1.)

liegt in Form eines farblosen Festkörpers vor; Smp. 85 ° C; Ausbeute: 96 g (91,7 %).

Auf analoge Weise werden die folgenden Verbindungen hergestellt

Tabelle 1:

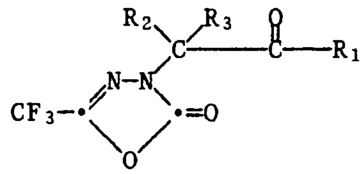

| Verbindung Nr. | $R_1$ | $R_2$ | $R_3$ | Physik. Daten |
|---|---|---|---|---|
| 1.1 | $CH_3$ | H | H | Smp. 85°C |
| 1.2 | i-$C_3H_7$ | H | H | Smp. 74-75°C |
| 1.3 | $C(CH_3)_3$ | H | H | Smp. 67°C |
| 1.4 | $C_6H_5$ | H | H | Smp. 100-102°C |
| 1.5 | $CH_3$ | $CH_3$ | H | Oel |
| 1.6 | $CH_3$ | $CH_3$ | $CH_3$ | Oel |
| 1.7 | $C_2H_5$ | H | H | Smp. 76-77°C |
| 1.8 | 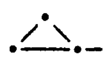 | H | H | Smp. 77-78°C |
| | H | H | H | |
| | $C_2H_5$ | $CH_3$ | H | |
| | n-$C_3H_7$ | H | H | |
| | 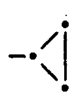 | $CH_3$ | H | |
| | H | $CH_3$ | H | |
| | i-$C_3H_7$ | $CH_3$ | H | |
| | i-$C_3H_7$ | $CH_3$ | $CH_3$ | |
| | $C(CH_3)_3$ | $CH_3$ | H | |
| | $CH_3$ | $C_2H_5$ | H | |
| | $CH_3$ | $C_2H_5$ | $CH_3$ | |

Tabelle 1 (Fortsetzung):

| Verbindung Nr. | $R_1$ | $R_2$ | $R_3$ | Physik. Daten |
|---|---|---|---|---|
| | 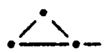 | $CH_3$ | $CH_3$ | |
| | 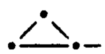 | $C_2H_5$ | $CH_3$ | |

Beispiel H.2: 2,3,4,5-Tetrahydro-3-oxo-4-amino-6-methyl-1,2,4-triazin

In 250 ml Hydrazinhydrat werden 210 g (1,0 Mol) 2-Oxo-5-trifluormethyl-2,3-dihydro-1,3,4-oxadiazol-3-aceton unter Kühlung eingetragen. Die resultierende klare braune Lösung wird nach 2-stündigem Rühren im Vakuum eingedampft und der Rückstand am Kieselgel (Methylenchlorid/methanol 9:1) chromatographiert. Das Lösungsmittel wird abgedampft und aus dem resultierenden Oel kristallisiert die Titelverbindung der Formel

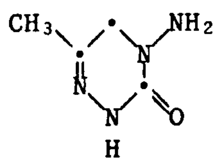

(Verbindung Nr. 2.1.)

nach Zusatz von Aether; Smp. 117-119 ° C; Ausbeute: 64 g (50 %).

Auf analoge Weise werden die folgenden Verbindungen hergestellt:

Tabelle 2:

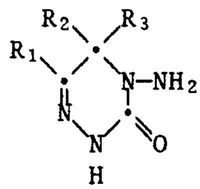

| Verbindung Nr. | $R_1$ | $R_2$ | $R_3$ | Smp. °C |
|---|---|---|---|---|
| 2.1 | $CH_3$ | H | H | 117-119 |
| 2.2 | $CH_3$ | $CH_3$ | H | 172-174 |
| 2.3 | $CH_3$ | $CH_3$ | $CH_3$ | 138-139 |
| 2.4 | $C_2H_5$ | H | H | 143-145 |
| 2.5 | $i\text{-}C_3H_7$ | H | H | 79-81 |
| 2.6 | $C(CH_3)_3$ | H | H | 148-150 |
| 2.7 | 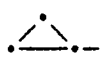 | H | H | 94-95 |
| 2.8 | $C_6H_5$ | H | H | 199-202 |
| 2.9 | $4\text{-}Cl\text{-}C_6H_4$ | H | H | 208-210 |
| | H | H | H | |
| | $CH_3$ | $C_2H_5$ | H | |
| | $C_2H_5$ | $CH_3$ | $CH_3$ | |
| | $C_2H_5$ | $CH_3$ | H | |
| | $n\text{-}C_3H_7$ | H | H | |
| | $n\text{-}C_3H_7$ | $CH_3$ | H | |
| | $n\text{-}C_3H_7$ | $CH_3$ | $CH_3$ | |
| | $i\text{-}C_3H_7$ | $CH_3$ | H | |
| | $C(CH_3)_3$ | $CH_3$ | H | |
| | $C(CH_3)_3$ | $CH_3$ | $CH_3$ | |

Tabelle 2 (Fortsetzung):

| Verbindung Nr. | $R_1$ | $R_2$ | $R_3$ | Smp. °C |
|---|---|---|---|---|
| | 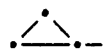 | $CH_3$ | $CH_3$ | |
| | 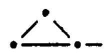 | $CH_3$ | H | |

Beispiel H.3: 2,3,4,5-Tetrahydro-3-oxo-4-[(pyridin-3-yl)-methylenamino]-6-methyl-1,2,4-triazin

32 g (0,25 Mol) 2,3,4,5-Tetrahydro-3-oxo-4-amino-6-methyl-1,2,4-triazin, gelöst in 250 ml Aethanol werden bei 60° C mit 26,8 g (0,25 Mol) von Pyridin-3-carbaldehyd und 1 Tropfen konzentrierter HCl versetzt. Nach halbstündigem Kochen unter Rückfluss wird das Reaktionsgut abgekühlt, der Festanteil abfiltriert, mit Aether gewaschen und getrocknet. Die Titelverbindung der Formel

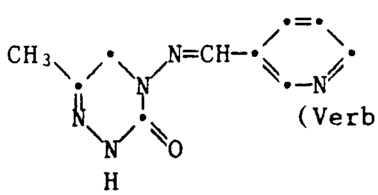

(Verbindung Nr. 3.1.)

liegt als farbloser Festkörper vor; Smp. 227-228° C; Ausbeute: 48 g (90 %).

Auf anaologe Weise werden die folgenden Verbindungen hergestellt:

Tabelle 3:

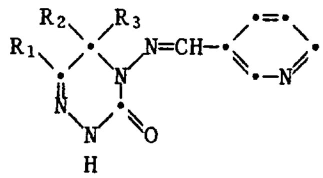

| Verbindung Nr. | $R_1$ | $R_2$ | $R_3$ | Physikalische Daten |
|---|---|---|---|---|
| 3.1 | $CH_3$ | H | H | Smp. 227-228°C |
| 3.2 | $CH_3$ | $CH_3$ | H | Smp. 139-141°C |
| 3.3 | $CH_3$ | $CH_3$ | $CH_3$ | Smp. 158°C |
| 3.4 | $C_2H_5$ | H | H | Smp. 223-224°C |
| 3.5 | $i-C_3H_7$ | H | H | Smp. 201-203°C |
| 3.6 | 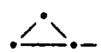 | H | H | Smp. 243-244°C |
| 3.7 | $C(CH_3)_3$ | H | H | Smp. 195-196°C |
| 3.8 | $C_6H_5$ | H | H | Smp. 263-264°C |
| 3.9 | $4-Cl-C_6H_4$ | H | H | Smp. 246-247°C |
| | H | H | H | |
| | H | $CH_3$ | H | |
| | H | $CH_3$ | $CH_3$ | |
| | $CH_3$ | $C_2H_5$ | H | |
| | $CH_3$ | $C_2H_5$ | $CH_3$ | |
| | $CH_3$ | $C_2H_5$ | $C_2H_5$ | |
| | $C_2H_5$ | $CH_3$ | H | |
| | $C_2H_5$ | $CH_3$ | $CH_3$ | |
| | $C_2H_5$ | $C_2H_5$ | H | |
| | $n-C_3H_7$ | H | H | |
| | $n-C_3H_7$ | $CH_3$ | H | |
| | $n-C_3H_7$ | $CH_3$ | $CH_3$ | |

Tabelle 3 (Fortsetzung):

| Verbindung Nr. | $R_1$ | $R_2$ | $R_3$ | Physik. Daten |
|---|---|---|---|---|
| | $n-C_3H_7$ | $C_2H_5$ | $CH_3$ | |
| | $i-C_3H_7$ | $CH_3$ | H | |
| | $i-C_3H_7$ | $CH_3$ | $CH_3$ | |
| | 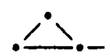 | $CH_3$ | H | |
| | 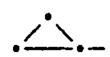 | $CH_3$ | $CH_3$ | |
| | $C(CH_3)_3$ | $CH_3$ | H | |
| | $C(CH_3)_3$ | $CH_3$ | $CH_3$ | |
| | $n-C_4H_9$ | H | H | |
| | $C_6H_5$ | $CH_3$ | H | |
| | $C_6H_5$ | $CH_3$ | $CH_3$ | |

Beispiel H.4: 2,3,4,5-Tetrahydro-3-oxo-[(pyridin-3-yl)-methylamino-6-isopropyl-1,2,4-triazin

In eine Suspension von 24,5 g (0,1 Mol) 2,3,4,5-Tetrahydro-3-oxo-4-[(pyridin-3-yl)-methylenamino]-6-isopropyl-1,2,4-triazin in 800 ml Methanol werden 37,8 g (1 Mol) Natriumborhydrid in Portionen eingetragen; das Reaktionsgut wird während einiger Stunden bei Raumtemperatur nachgerührt, und für 12 Stunden unter Rückfluss gekocht. Nach Verdampfen des Lösungsmittels wird der Rückstand mit Acetonitril verrührt und abfiltriert. Nach dem Eindampfen der Acetonitril-Lösung wird der Rückstand mit Aether verrührt und das Kristallisat abfiltriert. Die Titelverbindung der Formel

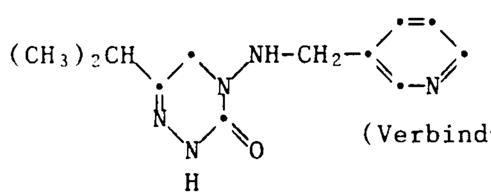

(Verbindung Nr. 4.1.)

liegt als farbloses Kristallpulver vor; Smp. 105-107 °C; Ausbeute: 12 g (49 %).

Auf analoge Weise werden die folgenden Verbindungen hergestellt:

Tabelle 4:

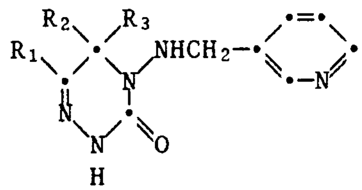

| Verbindung Nr. | $R_1$ | $R_2$ | $R_3$ | Physikalische Daten |
|---|---|---|---|---|
| 4.1 | $i-C_3H_7$ | H | H | Smp. 105-107°C |
| 4.2 | $CH_3$ | H | H | Smp. 161-163°C |
| 4.3 | $C(CH_3)_3$ | H | H | Smp. 162-164°C |
| 4.4 | $C_2H_5$ | H | H | Smp. 94-96°C |
| 4.5 | 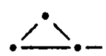 | H | H | Smp. 133-135°C |
| 4.6 | $CH_3$ | $CH_3$ | H | Smp. 35°C |
| 4.7 | $CH_3$ | $CH_3$ | $CH_3$ | Smp. 150°C |
| | H | H | H | |
| | H | $CH_3$ | H | |
| | $C_2H_5$ | $CH_3$ | H | |
| | $n-C_3H_7$ | H | H | |
| | $i-C_3H_7$ | $CH_3$ | H | |
| | $i-C_3H_7$ | $CH_3$ | $CH_3$ | |
| | 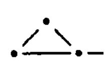 | $CH_3$ | H | |
| | 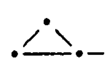 | $C_2H_5$ | $CH_3$ | |
| | $C(CH_3)_3$ | $CH_3$ | H | |
| | $C(CH_3)_3$ | $CH_3$ | $CH_3$ | |

Tabelle 4 (Fortsetzung):

| Verbindung Nr. | $R_1$ | $R_2$ | $R_3$ | Physik. Daten |
|---|---|---|---|---|
| | $C(CH_3)_3$ | $C_2H_5$ | H | |
| | $C_6H_5$ | H | H | |
| | $C_6H_5$ | $CH_3$ | H | |

Beispiel H.5: 2,3,4,5-Tetrahydro-3-oxo-4-[(pyridin-3-yl)-methylenamino]-6-methyl-1,2,4-triazin Hydrochlorid

21,7 g 2,3,4,5-Tetrahydro-3-oxo-4-[(pyridin-3-yl)-methylenamino]-6-methyl-1,2,4-triazin werden in 60 ml 2N-Salzsäure unter Erwärmen gelöst. Die heisse Lösung wird filtriert und abgekühlt. Der auskristallisierende Niederschlag wird abfiltriert, mit Alkohol und Aether gewaschen und im Vacuum getrocknet. Die Titelverbindung der Formel

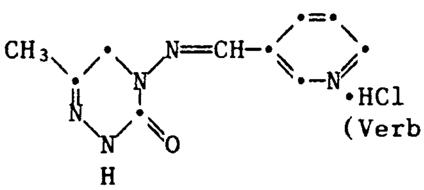

(Verbindung Nr. 5.1.)

liegt als farbloses Kristallpulver vor; Smp. 240-241 ° C u. Zers.; Ausbeute: 19 g (75 %).

Auf analoge Weise werden die folgenden Verbindungen hergestellt:

Tabelle 5:

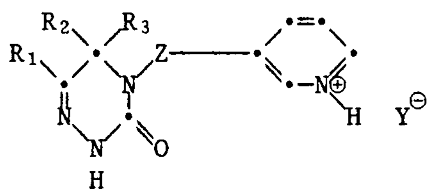

| Verbindung Nr. | $R_1$ | $R_2$ | $R_3$ | Z | $Y^{\ominus}$ | Smp. °C |
|---|---|---|---|---|---|---|
| 5.1 | $CH_3$ | H | H | -N=CH- | Cl | 240-241 |
| 5.2 | $CH_3$ | H | H | -N=CH- | $^{1}/_{2}SO_4$ | 237 |
| 5.3 | $C_2H_5$ | H | H | -N=CH- | Cl | 253 |
| 5.4 | $C_2H_5$ | H | H | -N=CH- | $^{1}/_{2}SO_4$ | 205 |
| 5.5 | $C_2H_5$ | H | H | -N=CH- | $NO_3$ | 181 |
| 5.6 | $CH_3$ | H | H | -N=CH- | $NO_3$ | 177 |
| 5.7 | $C_2H_5$ | H | H | -N=CH- | $CH_3SO_3$ | 224-225 |
| 5.8 | $CH_3$ | H | H | -N=CH- | $CF_3CO_2$ | 196 |
| 5.9 | $CH_3$ | H | H | -N=CH- | $^{1}/_{2}PO_4$ | 206-210 |
| 5.10 | $CH_3$ | H | H | -N=CH- | Oxals. | 218-219 |
| 5.11 | $(CH_3)_3C$ | H | H | -N=CH- | Cl | 229-230 |
| 5.12 | 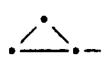 | H | H | -N=CH- | $NO_3$ | 229-230 |
| 5.13 | 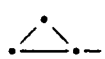 | H | H | -N=CH- | Cl | 250 |
| 5.14 | 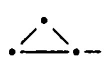 | H | H | -N=CH- | $CF_3CO_2$ | 196-198 |
| 5.15 | 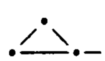 | H | H | -N=CH- | Oxals. | 220 |
| 5.16 | 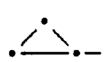 | H | H | -N=CH- | $^{1}/_{2}SO_4$ | 210 |
| 5.17 | 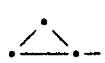 | H | H | -N=CH- | $^{1}/_{2}PO_4$ | 219 |

2. Formulierungsbeispiele

Formulierungen für Wirkstoffe der Formel I resp. Kombinationen dieser Wirkstoffe mit andern Insektiziden oder Akariziden (% = Gewichtsprozent):

| F1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

| F2. Emulsions-Konzentrat | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

| F4. Extruder-Granulat | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| F5. Umhüllungs-Granulat | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| F6. Suspensions-Konzentrat | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel B.1: Wirkung gegen Aedes aegypti (Larven)

Auf die Oberfläche von 150 ml Wasser, das sich in einem Behälter befindet, wird so viel einer 0,1%igen acetonischen Lösung des Wirkstoffes pipettiert, dass eine Konzentration von 400 ppm erhalten wird. Nach Verdunsten des Acetons wird der Behälter mit 30 bis 40 2-tägigen Aedes-Larven beschickt. Nach 2 und 7 Tagen wird die Mortalität geprüft.

Verbindungen gemäss den Beispielen H.3 bis H.5 zeigen gute Wirkung im obigen Test.

Beispiel B.2: Kontaktwirkung auf Aphis craccivora

In Töpfen angezogene 4-5 Tage alte Erbsenkeimlinge (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 12,5 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22 °C und einer rel. Luftfeuchtigkeit von etwa 55 % durchgeführt.

Verbindungen gemäss den Beispielen H.3 bis H.5 zeigen gute Wirkung in diesem Test.

Beispiel B.3: Systemische Wirkung auf Aphis craccivora

Bewurzelte Bohnenpflanzen werden in Töpfe, welche 600 ccm Erde enthalten, verpflanzt. Anschliessend giesst man 50 ml einer Zubereitung der zu prüfenden Verbindungen (erhalten aus einem 25 %igen Spritzpulver) in einer Konzentration von 400 ppm direkt auf die Erde in den Töpfen.

Nach 24 Stunden werden auf die oberirdischen Pflanzenteile Blattläuse der Spezies Aphis craccivora gesetzt und die Pflanzen mit einem Plastikzylinder überstülpt, um die Läuse vor einer eventuellen direkten Kontakt- oder Gaswirkung der Testsubstanz zu schützen.

Die Auswertung der erzielten Abtötung erfolgt 48 und 72 Stunden nach Versuchsbeginn. Pro Testsubstanz werden zwei Pflanzen, je eine in einem separaten Topf, verwendet. Der Versuch wird bei 25 °C und ca. 70 % relativer Luftfeuchtigkeit durchgeführt.
Verbindungen gemäss den Beispielen H.3 bis H.5 zeigen gute Wirkung in diesem Test.

Beispiel B.4: Kontaktwirkung auf Myzus persicae, Direktspraytest

Peperonipflanzen (im 6-Blattstadium, eingetopft) werden 4 Tage vor der Behandlung mit einer Population von Myzus Persicae (R-Stamm) invertiert, indem man 2-3 cm lange, mit Blattläusen gut besiedelte Erbsenkeimlinge auf die Peperonipflanzen legt. Sobald die Erbsenkeimlinge zu vertrocknen beginnen, wandern die Blattläuse auf die Versuchspflanzen (Peperoni) über. Die so behandelten Pflanzen werden 24 Stunden später mit einer, aus 25%-igem Spritzpulver hergestellten wässrigen Suspension enthaltend 100 ppm der zu prüfenden Verbindung bis zur Tropfnässe direkt besprüht. Man verwendet pro Testsubstanz vier Pflanzen. Eine Auswertung der erzielten Abtötungsrate erfolgt 7 Tage nach Applikation. Der Versuch wird bei 21-22 °C und etwa 60 % relativer Luftfeuchtigkeit durchgeführt.

Die Verbindungen gemäss den Beispielen H.3 bis H.5 zeigen in diesem Test 80-100 %ige Abtötung.

Beispiel B.5: Dauerwirkungstests Myzus persicae

Peperonipflanzen (im 6-Blattstadium, eingetopft) werden mit den Versuchslösungen mittels Sprayapplikation behandelt, 2 Tage nach der Behandlung werden die Versuchspflanzen mit einer Population von Myzus Persicae (R-Stamm), wie in Beispiel B4 beschrieben, besiedelt. Die Bonitur erfolgt 5 Tage nach der Besiedlung auf % Mortalität.

Die Verbindungen gemäss den Beispielen H.3 bis H.5 zeigen bei einer Konzentration von 100 ppm 50-100 %ige Abtötung.

**Patentansprüche**

**Patentansprüche für folgende Vertragsstaaten : AT, BE, CH, DE, FR, GB, IT, LI, NL**

**1.** Verbindungen der Formel I

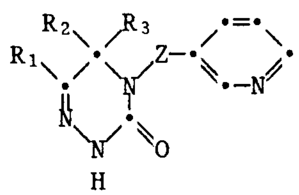

(I),

worin

$R_1$ Wasserstoff, $C_1$-$C_{12}$-Alkyl, $C_3$-$C_6$-Cycloalkyl, $C_1$-$C_4$-Alkoxy-$C_1$-$C_6$-alkyl, $C_1$-$C_2$-Halogenalkyl, Phenyl, Benzyl, Phenäthyl, Phenpropyl, Phenbutyl, Phenpentyl oder einen ein- oder zweifach durch Halogen, $C_1$-$C_5$-Alkyl, $C_1$-$C_2$-Halogenalkyl, Methoxy und/oder Aethoxy substituierten Phenyl-, Benzyl-, Phenäthyl-, Phenpropyl-, Phenbutyl- oder Phenpentylrest,

$R_2$ Wasserstoff, $C_1$-$C_6$-Alkyl, $C_3$-$C_6$-Cycloalkyl, unsubstituiertes oder durch $C_1$-$C_{12}$-Alkyl, Halogen oder $C_1$-$C_{12}$-Halogenalkyl substituiertes Phenyl oder $R_1$ und $R_2$ zusammen einen gesättigten oder ungesättigten 3- bis 7-gliedrigen Carbocyclus bilden,

$R_3$ Wasserstoff oder $C_1$-$C_6$-Alkyl bedeuten und

Z für -N = CH- oder -NH-$CH_2$- steht,

sowie ihre Salze mit organischen oder anorganischen Säuren.

**2.** Verbindungen der Formel I gemäss Anspruch 1, worin $R_1$ Wasserstoff, $C_1$-$C_6$-Alkyl, $C_3$-$C_5$-Cycloalkyl, Phenyl oder ein- oder zweifach durch Halogen, $C_1$-$C_3$-Alkyl, Methoxy oder Aethoxy substituiertes Phenyl; $R_2$ und $R_3$ je Wasserstoff oder $C_1$-$C_4$-Alkyl bedeuten und Z für -N = CH- oder -NH-$CH_2$- steht.

**3.** Verbindungen der Formel I, gemäss Anspruch 2, worin $R_1$ für Wasserstoff, $C_1$-$C_4$-Alkyl, Cyclopropyl oder Phenyl; $R_2$ für Wasserstoff, Methyl oder Aethyl; $R_3$ für Wasserstoff oder Methyl; und Z für -N = CH-stehen.

**4.** Verbindungen der Formel I, gemäss Anspruch 2, worin $R_1$ für Wasserstoff, $C_1$-$C_4$-Alkyl, Cyclopropyl oder Phenyl; $R_2$ für Wasserstoff, Methyl oder Aethyl; $R_3$ für Wasserstoff oder Methyl; und Z für -NH-$CH_2$-stehen.

**5.** Eine Verbindung gemäss Anspruch 3 der Formel

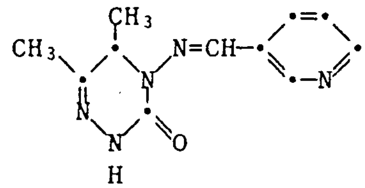

,

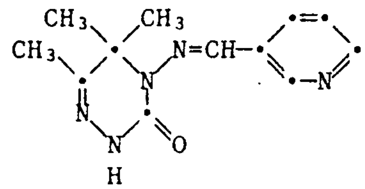

,

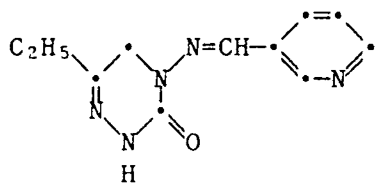

,

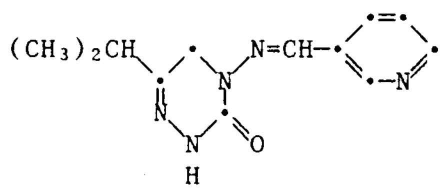

,

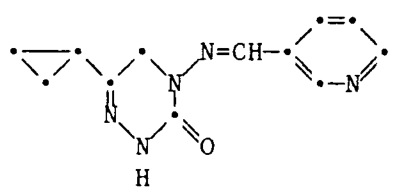

,

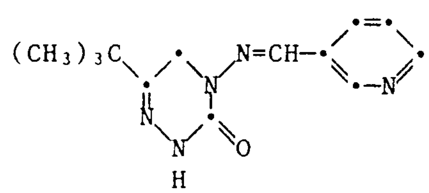

,

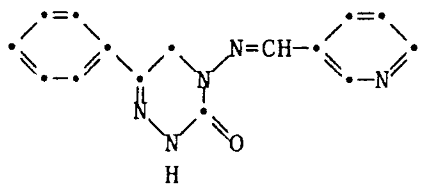

,

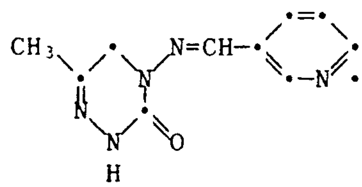 HCl ,

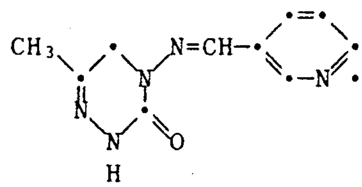 1/2 $H_2SO_4$ ,

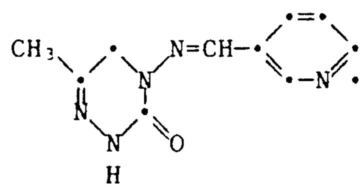 $HNO_3$ ,

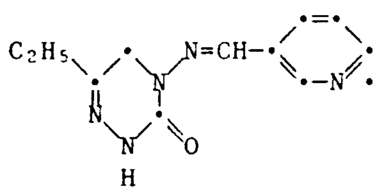 HCl ,

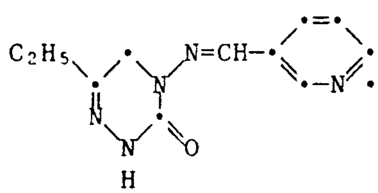 1/2 $H_2SO_4$ oder

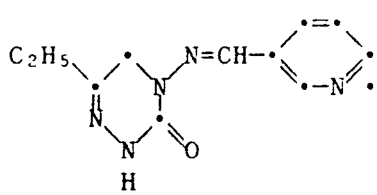 $HNO_3$ .

**6.** Die Verbindung gemäss Anspruch 3 der Formel

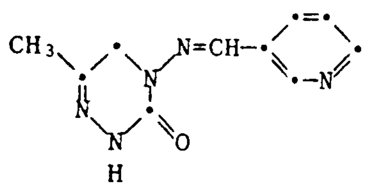

.

**7.** Eine Verbindung gemäss Anspruch 4 der Formel

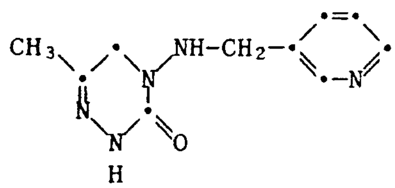

,

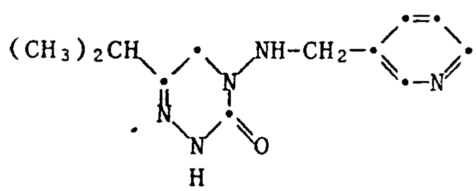

,

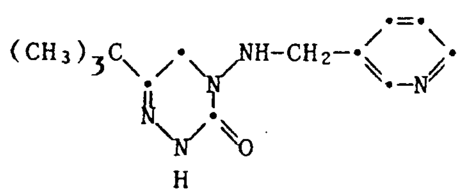

,

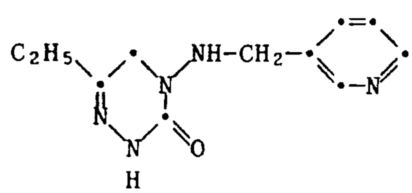

oder

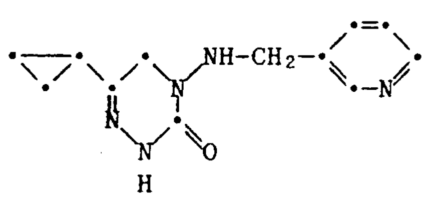

.

**8.** Verfahren zur Herstellung einer Verbindung der Formel

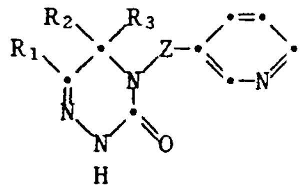

(I),

worin Z, $R_1$, $R_2$ und $R_3$ die in Anspruch 1 angegebene Bedeutung haben, dadurch gekennzeichnet, dass man

A. ein Aninotriazinon der Formel II

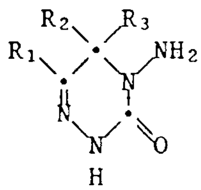

(II),

worin $R_1$, $R_2$ und $R_3$ die in Formel I angegebene Bedeutung haben, mit dem Aldehyd der Formel III

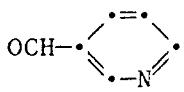

(III)

umsetzt und gegebenenfalls

B. das erhaltene Pyridyl-methylenamino-triazinon durch selektive Reduktion in das Pyridyl-methylamino-triazinon überführt.

**9.** Schädlingsbekämpfungsmittel, welches als aktive Komponente eine Verbindung der Formel I gemäss Anspruch 1 oder eines ihrer Salze mit einer organischen oder anorganischen Säure zusammen mit geeigneten Trägern und/oder Zuschlagstoffen enthält.

**10.** Schädlingsbekämpfungsmittel gemäss Anspruch 9, welches als aktive Komponente eine Verbindung gemäss einem der Ansprüche 2 bis 7 enthält.

**11.** Verwendung einer Verbindung der Formel I gemäss Anspruch 1 oder eines ihrer Salze mit einer organischen oder anorganischen Säure zur Bekämpfung von Schädlingen an Pflanzen.

**12.** Verwendung gemäss Anspruch 11 zur Bekämpfung von Insekten und vertretern der Ordnung Akarina.

**13.** Verwendung gemäss Anspruch 12 zur Bekämpfung von pflanzenschädigenden Insekten.

**14.** Verwendung gemäss Anspruch 13 zur Bekämpfung von saugenden Insekten.

**15.** Verfahren zum Bekämpfen von Schädlingen an Pflanzen, dadurch gekennzeichnet, dass man die Schädlinge in ihren verschiedenen Entwicklungsstadien mit einer Verbindung der Formel I gemäss Anspruch 1 oder einem ihrer Salze mit einer organischen oder anorganischen Säure in Kontakt bringt.

**16.** Verbindungen der Formel IIa

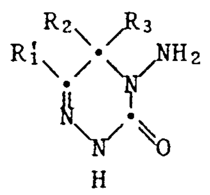

(IIa),

worin

| | |
|---|---|
| $R_1'$ | Wasserstoff, $C_1$-$C_{12}$-Alkyl, $C_3$-$C_6$-Cycloalkyl, $C_1$-$C_4$-Alkoxy-$C_1$-$C_6$-alkyl, $C_1$-$C_2$-Halogenalkyl, Benzyl, Phenäthyl, Phenpropyl, Phenbutyl, Phenpentyl oder einen ein- oder zweifach durch Halogen, $C_1$-$C_5$-Alkyl, $C_1$-$C_2$-Halogenalkyl, Methoxy und/oder Aethoxy substituierten Phenyl-, Benzyl-, Phenäthyl-, Phenpropyl-, Phenbutyl- oder Phenpentylrest und |
| $R_2$ und $R_3$ | die für Formel I in Anspruch 1 angegebene Bedeutung haben. |

**17.** Verbindungen der Formel IIa gemäss Anspruch 16, worin $R_1'$ Wasserstoff, $C_1$-$C_6$-Alkyl, $C_3$-$C_5$-Cycloalkyl oder ein- oder zweifach durch Halogen, $C_1$-$C_3$-Alkyl, Methoxy oder Aethoxy substituiertes Phenyl; und $R_2$ und $R_3$ je Wasserstoff oder $C_1$-$C_4$-Alkyl bedeuten.

**18.** Verbindungen der Formel IIa gemäss Anspruch 17, worin $R_1'$ für Wasserstoff, $C_1$-$C_4$-Alkyl oder Cyclopropyl; $R_2$ für Wasserstoff, Methyl oder Aethyl; und $R_3$ für Wasserstoff oder Methyl stehen.

**19.** Die Verbindungen gemäss Anspruch 18 der Formeln

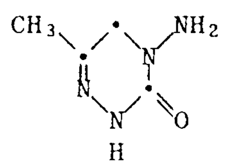

,

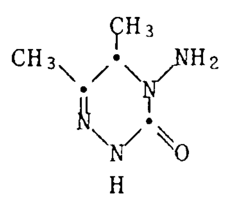

,

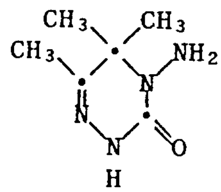

,

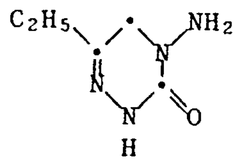

,

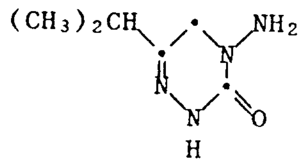

,

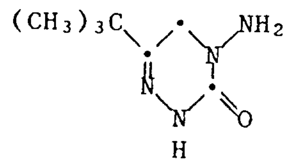

und

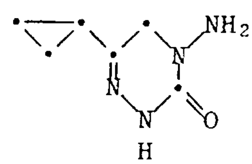

.

**20.** Verbindungen der Formel IV

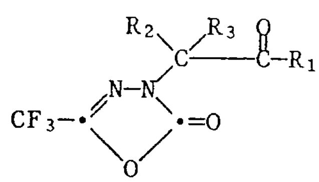

(IV),

worin $R_1$, $R_2$ und $R_3$ die für Formel I in Anspruch 1 angegebene Bedeutung haben.

**21.** Verbindungen der Formel IV gemäss Anspruch 20, worin $R_1$ Wasserstoff, $C_1$-$C_6$-Alkyl, $C_3$-$C_5$-Cycloalkyl, Phenyl oder ein- oder zweifach durch Halogen, $C_1$-$C_3$-Alkyl, Methoxy oder Aethoxy substituiertes Phenyl; und $R_2$ und $R_3$ je Wasserstoff oder $C_1$-$C_4$-Alkyl bedeuten.

**22.** Verbindungen der Formel IV gemäss Anspruch 21, worin $R_1$ für Wasserstoff, $C_1$-$C_4$-Alkyl oder Cyclopropyl; $R_2$ für Wasserstoff, Methyl oder Aethyl; und $R_3$ für Wasserstoff oder Methyl stehen.

**23.** Die Verbindungen gemäss Anspruch 22 der Formeln

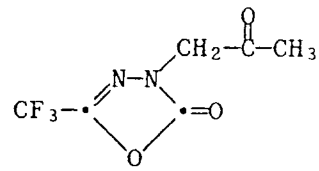

,

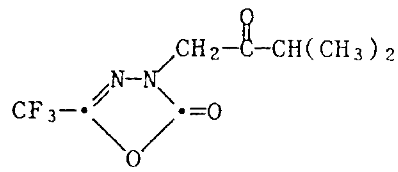

,

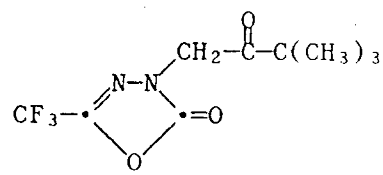

,

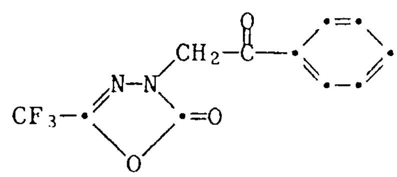

,

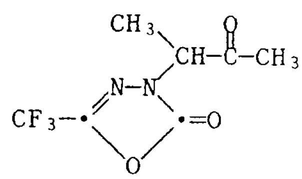

,

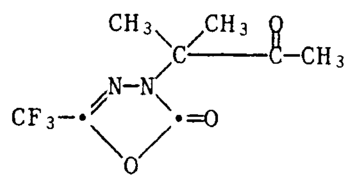

,

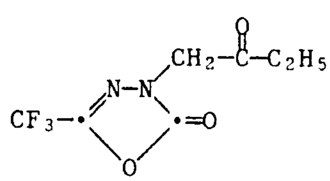

und

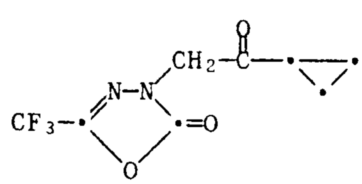

.

**Patentansprüche für folgenden Vertragsstaat : ES**

1. Verfahren zur Herstellung einer Verbindung der Formel

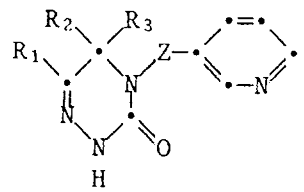

(I),

worin

$R_1$ Wasserstoff, $C_1$-$C_{12}$-Alkyl, $C_3$-$C_6$-Cycloalkyl, $C_1$-$C_4$-Alkoxy-$C_1$-$C_6$-alkyl, $C_1$-$C_2$-Halogenalkyl, Phenyl, Benzyl, Phenäthyl, Phenpropyl, Phenbutyl, Phenpentyl oder einen ein- oder zweifach durch Halogen. $C_1$-$C_5$-Alkyl, $C_1$-$C_2$-Halogenalkyl, Methoxy und/oder Aethoxy substituierten Phenyl-, Benzyl-, Phenäthyl-, Phenpropyl-, Phenbutyl- oder Phenpentylrest,

$R_2$ Wasserstoff, $C_1$-$C_6$-Alkyl, $C_3$-$C_6$-Cycloalkyl, unsubstituiertes oder durch $C_1$-$C_{12}$-Alkyl, Halogen oder $C_1$-$C_{12}$-Halogenalkyl substituiertes Phenyl oder $R_1$ und $R_2$ zusammen einen gesättigten oder ungesättigten 3- bis 7-gliedrigen Carbocyclus bilden,

$R_3$ Wasserstoff oder $C_1$-$C_6$-Alkyl bedeuten und

Z für -N = CH- oder -NH-$CH_2$- steht,

sowie ihre Salze mit organischen oder anorganischen Säuren,
dadurch gekennzeichnet, dass man

A. ein Aminotriazinon der Formel

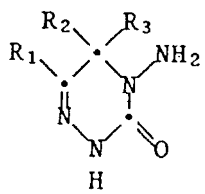

(II),

worin $R_1$, $R_2$ und $R_3$ die in Formel I angegebene Bedeutung haben,
mit dem Aldehyd der Formel

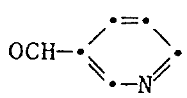

(III)

umsetzt und gegebenenfalls

B. das erhaltene Pyridyl-methylenamino-triazinon durch selektive Reduktion in das Pyridyl-methylamino-triazinon überführt.

2. Verfahren gemäss Anspruch 1 zur Herstellung einer Verbindung der Formel I, worin $R_1$ Wasserstoff, $C_1$-$C_6$-Alkyl, $C_3$-$C_5$-Cycloalkyl, Phenyl oder ein- oder zweifach durch Halogen, $C_1$-$C_3$-Alkyl, Methoxy oder Aethoxy substituiertes Phenyl; $R_2$ und $R_3$ je Wasserstoff oder $C_1$-$C_4$-Alkyl bedeuten und Z für -N = CH- oder -NH-$CH_2$- steht.

3. Verfahren gemäss Anspruch 2 zur Herstellung einer Verbindung der Formel I, worin $R_1$ für Wasserstoff, $C_1$-$C_4$-Alkyl, Cyclopropyl oder Phenyl; $R_2$ für Wasserstoff, Methyl oder Aethyl; $R_3$ für Wasserstoff oder Methyl; und Z für -N = CH-stehen.

4. Verfahren gemäss Anspruch 2 zur Herstellung einer Verbindung der Formel I, worin $R_1$ für Wasserstoff, $C_1$-$C_4$-Alkyl, Cyclopropyl oder Phenyl; $R_2$ für Wasserstoff, Methyl oder Aethyl; $R_3$ für Wasserstoff oder Methyl; und Z für -NH-$CH_2$-stehen.

**5.** Verfahren gemäss Anspruch 3 zur Herstellung einer Verbindung der Formel

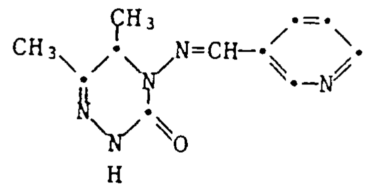

,

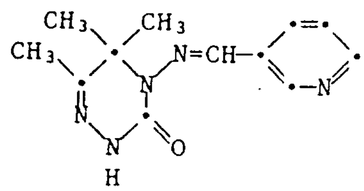

,

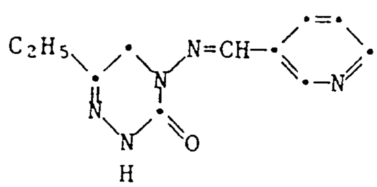

,

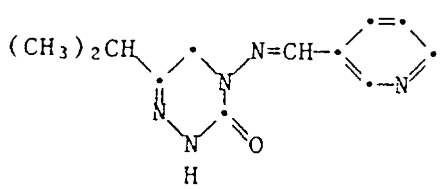

,

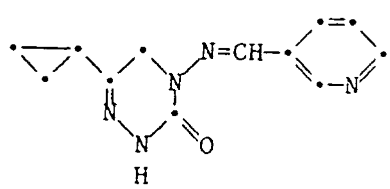

,

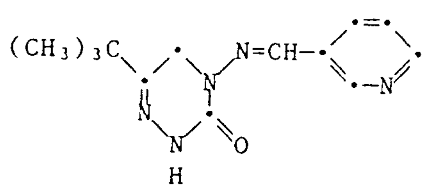

,

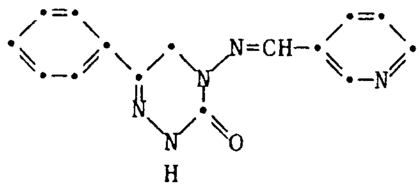

,

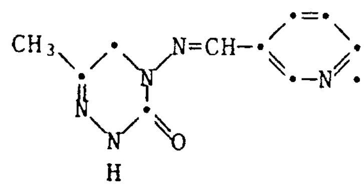

$\cdot$ HCl ,

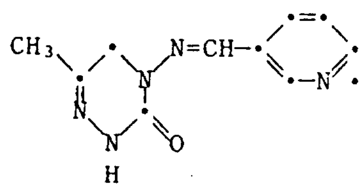

$\cdot$ 1/2 $H_2SO_4$ ,

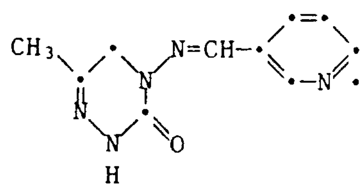

$\cdot$ $HNO_3$ ,

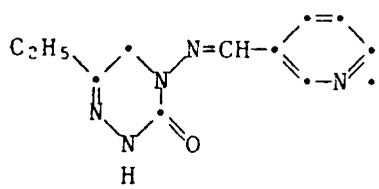

$\cdot$ HCl ,

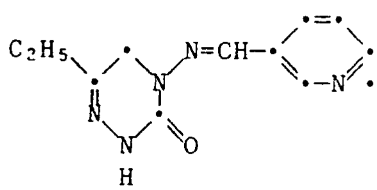

$\cdot$ 1/2 $H_2SO_4$ oder

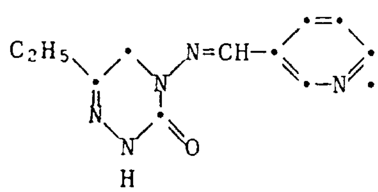

$\cdot$ $HNO_3$ .

6. Verfahren gemäss Anspruch 3 zur Herstellung einer Verbindung der Formel

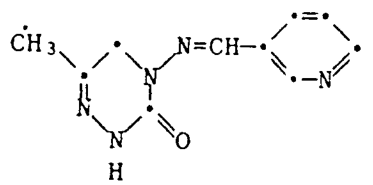

.

7. Verfahren gemäss Anspruch 4 zur Herstellung einer Verbindung der Formel

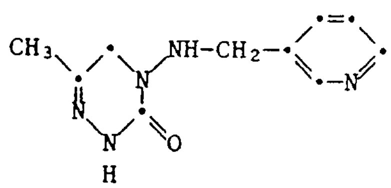

,

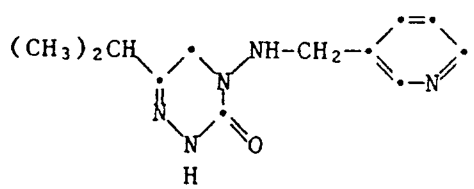

,

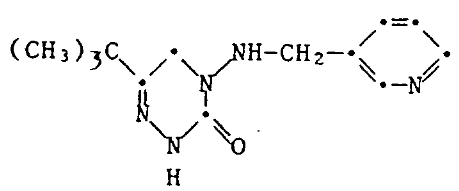

,

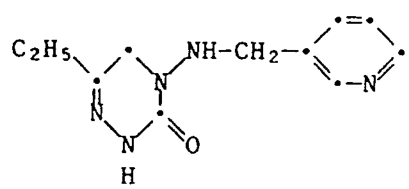

oder

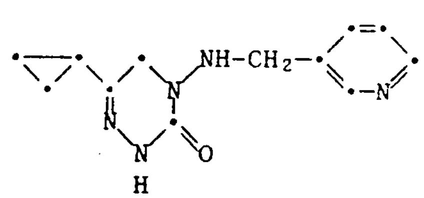

.

8. Schädlingsbekämpfungsmittel, welches als aktive Komponente eine Verbindung der Formel I, erhältlich gemäss Anspruch 1, oder eines ihrer Salze mit einer organischen oder anorganischen Säure zusammen mit geeigneten Trägern und/oder Zuschlagstoffen enthält.

9. Schädlingsbekämpfungsmittel gemäss Anspruch 8, welches als aktive Komponente eine Verbindung, erhältlich gemäss einem der Ansprüche 2 bis 7, enthält.

**10.** Verwendung einer Verbindung der Formel I, erhältlich gemäss Anspruch 1, oder eines ihrer Salze mit einer organischen oder anorganischen Säure zur Bekämpfung von Schädlingen an Pflanzen.

**11.** Verwendung gemäss Anspruch 10 zur Bekämpfung von Insekten und vertretern der Ordnung Akarina.

**12.** Verwendung gemäss Anspruch 11 zur Bekämpfung von pflanzenschädigenden Insekten.

**13.** Verwendung gemäss Anspruch 12 zur Bekämpfung von saugenden Insekten.

**14.** Verfahren zum Bekämpfen von Schädlingen an Pflanzen, dadurch gekennzeichnet, dass man die Schädlinge in ihren verschiedenen Entwicklungsstadien mit einer Verbindung der Formel I, erhältlich gemäss Anspruch 1, oder einem ihrer Salze mit einer organischen oder anorganischen Säure in Kontakt bringt.

**15.** Verfahren zur Herstellung einer Verbindung der Formel

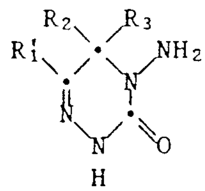

(IIa),

worin

$R_1'$ Wasserstoff, $C_1$-$C_{12}$-Alkyl, $C_3$-$C_6$-Cycloalkyl, $C_1$-$C_4$-Alkoxy-$C_1$-$C_6$-alkyl, $C_1$-$C_2$-Halogenalkyl, Benzyl, Phenäthyl, Phenpropyl, Phenbutyl, Phenpentyl oder einen ein- oder zweifach durch Halogen, $C_1$-$C_5$-Alkyl, $C_1$-$C_2$-Halogenalkyl, Methoxy und/oder Aethoxy substituierten Phenyl-, Benzyl-, Phenäthyl-, Phenpropyl-, Phenbutyl- oder Phenpentylrest bedeutet und

$R_2$ und $R_3$ die für Formel I in Anspruch 1 angegebene Bedeutung haben,

dadurch gekennzeichnet, dass man eine Verbindung der Formel

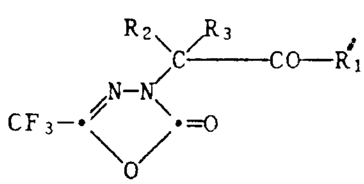

(IV) ,

worin $R_1'$ , $R_2$ und $R_3$ die für die Formel IIa angegebenen Bedeutungen aufweisen, mit Hydrazinhydrat umsetzt.

**16.** Verfahren gemäss Anspruch 15 zur Herstellung einer Verbindung der Formel IIa, worin $R_1'$ Wasserstoff, $C_1$-$C_6$-Alkyl, $C_3$-$C_5$-Cycloalkyl oder ein- oder zweifach durch Halogen, $C_1$-$C_3$-Alkyl, Methoxy oder Aethoxy substituiertes Phenyl; und $R_2$ und $R_3$ je Wasserstoff oder $C_1$-$C_4$-Alkyl bedeuten.

**17.** Verfahren gemäss Anspruch 16 zur Herstellung einer Verbindung der Formel IIa, worin $R_1'$ für Wasserstoff, $C_1$-$C_4$-Alkyl oder Cyclopropyl; $R_2$ für Wasserstoff, Methyl oder Aethyl; und $R_3$ für Wasserstoff oder Methyl stehen.

**18.** Verfahren gemäss Anspruch 17 zur Herstellung einer Verbindung der Formel

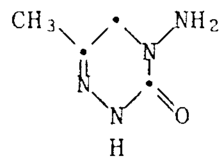

,

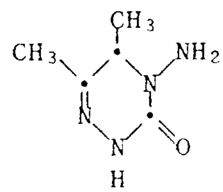

,

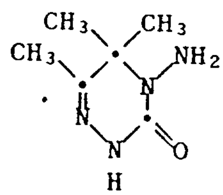

,

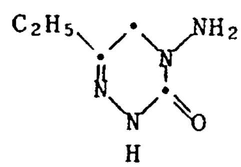

,

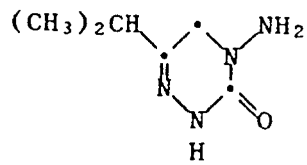

,

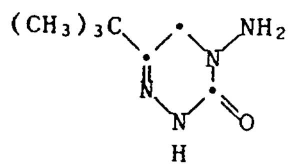

oder

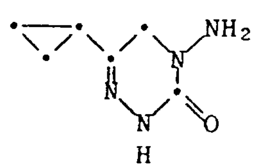

.

**19.** Verfahren zur Herstellung einer Verbindung der Formel

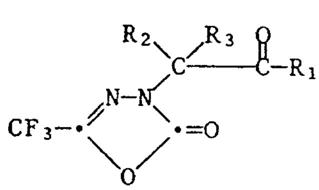

(IVa),

worin $R_1$, $R_2$ und $R_3$ die für Formel I in Anspruch angegebene Bedeutung haben, dadurch gekennzeichnet, dem man eine Verbindung der Formel

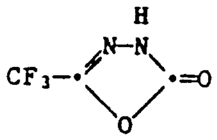

(V)

mit einer Verbindung der Formel

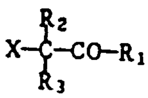

(VI),

worin $R_1$, $R_2$ und $R_3$ die für Formel I, Anspruch 1, angegebene Bedeutung haben und X für Halogen steht, umsetzt.

**20.** Verfahren gemäss Anspruch 19 zur Herstellung einer Verbindung der Formel IVa, worin $R_1$ Wasserstoff, $C_1$-$C_6$-Alkyl, $C_3$-$C_5$-Cycloalkyl, Phenyl oder ein- oder zweifach durch Halogen, $C_1$-$C_3$-Alkyl, Methoxy oder Aethoxy substituiertes Phenyl; und $R_2$ und $R_3$ je Wasserstoff oder $C_1$-$C_4$-Alkyl bedeuten.

**21.** Verfahren gemäss Anspruch 20 zur Herstellung einer Verbindung der Formel IVa, worin $R_1$ für Wasserstoff, $C_1$-$C_4$-Alkyl oder Cyclopropyl; $R_2$ für Wasserstoff, Methyl oder Aethyl; und $R_3$ für Wasserstoff oder Methyl stehen.

**22.** Verfahren gemäss Anspruch 21 zur Herstellung einer Verbindung der Formel

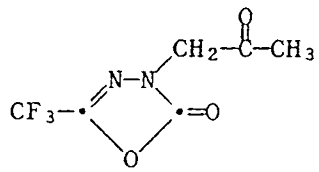

,

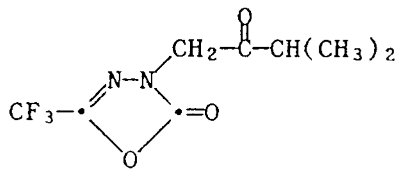

,

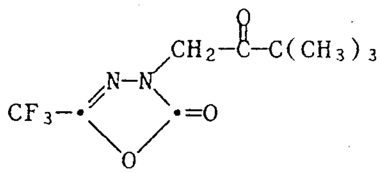

,

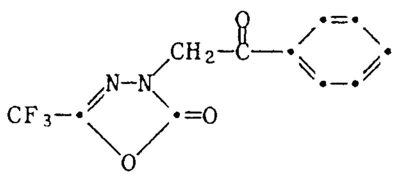

,

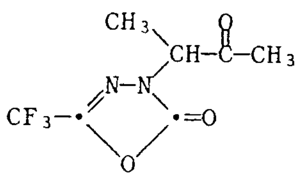

,

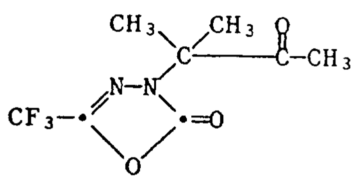

,

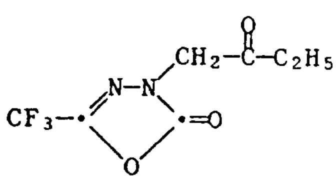

oder

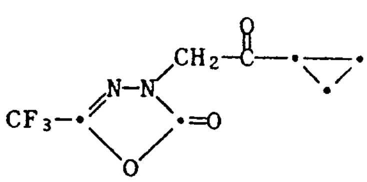

.

## Claims

**Claims for the following Contracting States : AT, BE, CH, DE, FR, GB, IT, LI, NL**

**1.** A compound of formula I

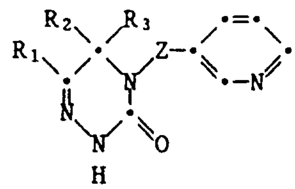

(I)

wherein
$R_1$ is hydrogen, $C_1$-$C_{12}$alkyl, $C_3$-$C_6$cycloalkyl, $C_1$-$C_4$-alkoxy-$C_1$-$C_6$alkyl, $C_1$-$C_2$haloalkyl, phenyl, benzyl, phenethyl, phenpropyl, phenbutyl or phenpentyl, or a phenyl, benzyl, phenethyl, phenpropyl, phenbutyl or phenpentyl radical that is mono- or di-substituted by halogen, $C_1$-$C_5$alkyl, $C_1$-$C_2$haloalkyl, methoxy and/or by ethoxy,
$R_2$ is hydrogen, $C_1$-$C_6$alkyl or $C_3$-$C_6$cycloalkyl, or is phenyl that is unsubstituted or substituted by $C_1$-$C_{12}$-alkyl, halogen or by $C_1$-$C_{12}$haloalkyl, or $R_1$ and $R_2$ together form a saturated or unsaturated 3- to 7-membered carbocycle,
$R_3$ is hydrogen or $C_1$-$C_6$alkyl, and
Z is -N=CH- or -NH-$CH_2$-,
or a salt thereof with an organic or inorganic acid.

**2.** A compound of formula I according to claim 1, wherein $R_1$ is hydrogen, $C_1$-$C_6$alkyl, $C_3$-$C_5$cycloalkyl, phenyl or phenyl that is mono- or di-substituted by halogen, $C_1$-$C_3$alkyl, methoxy or by ethoxy; each of $R_2$ and $R_3$ is hydrogen or $C_1$-$C_4$alkyl; and Z is -N=CH- or -NH-$CH_2$-.

**3.** A compound of formula I according to claim 2, wherein $R_1$ is hydrogen, $C_1$-$C_4$alkyl, cyclopropyl or phenyl; $R_2$ is hydrogen, methyl or ethyl; $R_3$ is hydrogen or methyl; and Z is -N=CH-.

**4.** A compound of formula I according to claim 2, wherein $R_1$ is hydrogen, $C_1$-$C_4$alkyl, cyclopropyl or phenyl; $R_2$ is hydrogen, methyl or ethyl; $R_3$ is hydrogen or methyl; and Z is -NH-$CH_2$-.

**5.** A compound according to claim 3 of formula

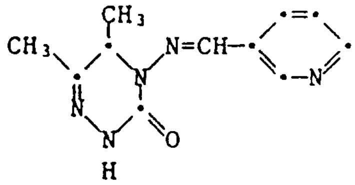

,

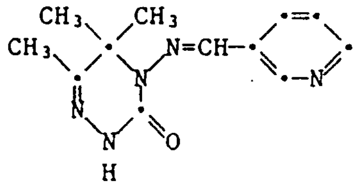

,

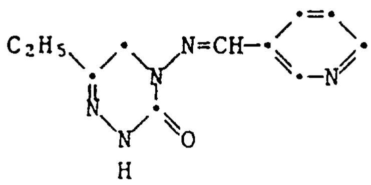

,

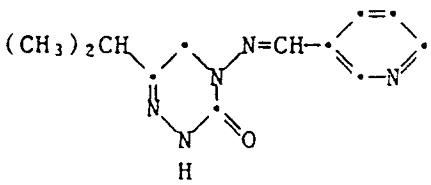

,

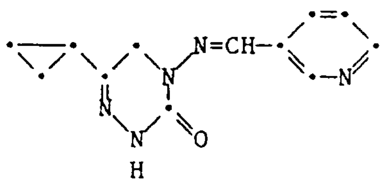

,

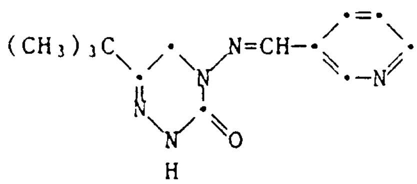

,

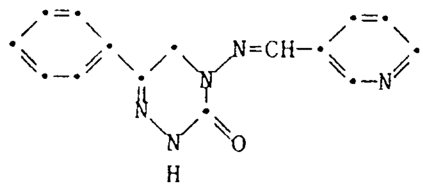

,

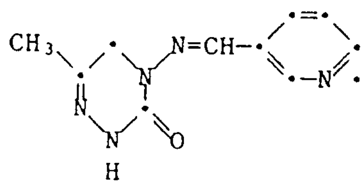

HCl ,

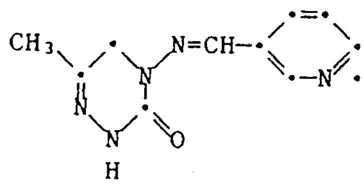

1/2 $H_2SO_4$ ,

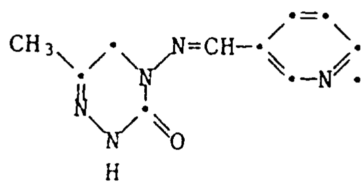

$HNO_3$ ,

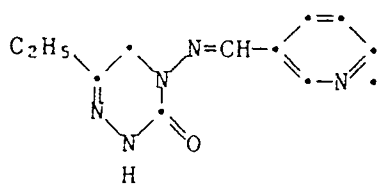

HCl ,

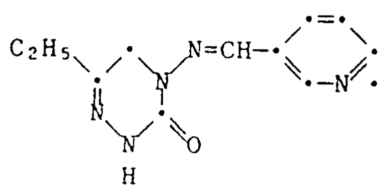

1/2 $H_2SO_4$ or

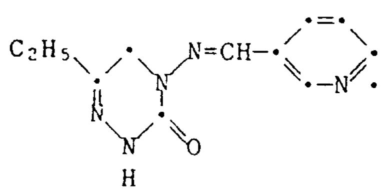

$HNO_3$ .

6. A compound according to claim 3 of formula

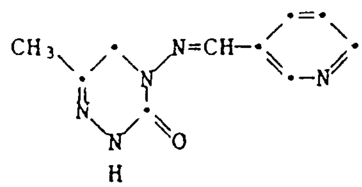

.

7. A compound according to claim 4 of formula

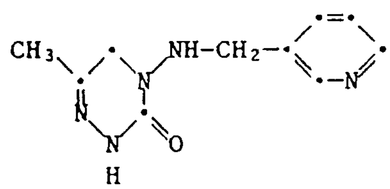

,

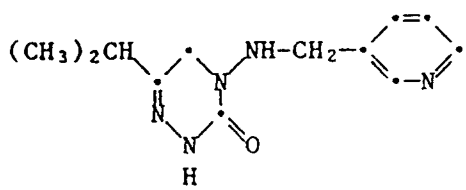

,

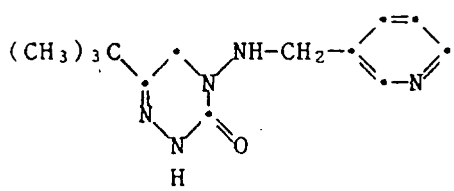

,

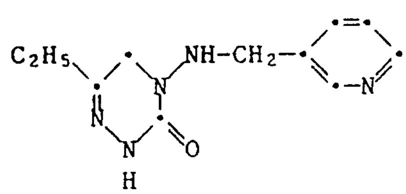

or

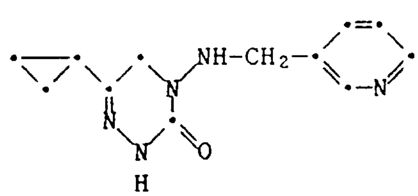

.

**8.** A process for the preparation of a compound of formula

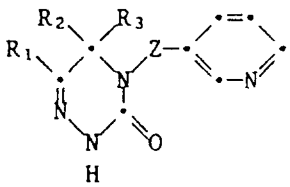

(I)

wherein Z, $R_1$, $R_2$ and $R_3$ are as defined in claim 1, which process comprises
A. reacting an aminotriazinone of formula II

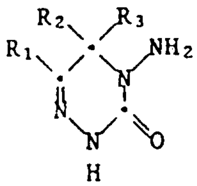

(II),

wherein $R_1$, $R_2$ and $R_3$ are as defined in formula I, with an aldehyde of formula III

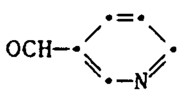

(III)

and, if desired,
B. converting the resulting pyridyl-methyleneaminotriazinone by selective reduction into pyridyl-methylamino-triazinone.

**9.** A pesticidal composition which comprises as active component a compound of formula I according to claim 1 or a salt thereof with an organic or inorganic acid, together with suitable carriers and/or adjuvants.

**10.** A pesticidal composition according to claim 9, which comprises as active component a compound according to any one of claims 2 to 7.

**11.** The use of a compound of formula I according to claim 1, or of a salt thereof with an organic or inorganic acid, for controlling pests on plants.

**12.** The use according to claim 11 for controlling insects and representatives of the order Acarina.

**13.** The use according to claim 12 for controlling plant-destructive insects.

**14.** The use according to claim 13 for controlling sucking insects.

**15.** A method of controlling pests on plants, which comprises bringing the pests in their various stages of development into contact with a compound of formula I according to claim 1 or with a salt thereof with an organic or inorganic acid.

**16.** A compound of formula IIa

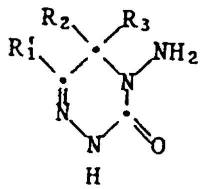

(IIa)

wherein
$R_1'$ is hydrogen, $C_1$-$C_{12}$alkyl, $C_3$-$C_6$cycloalkyl, $C_1$-$C_4$-alkoxy-$C_1$-$C_6$alkyl, $C_1$-$C_2$haloalkyl, benzyl, phenethyl, phenpropyl, phenbutyl or phenpentyl, or a phenyl, benzyl, phenethyl, phenpropyl, phenbutyl or phenpentyl radical that is mono- or di-substituted by halogen, $C_1$-$C_5$alkyl,$C_1$-$C_2$haloalkyl, methoxy and/or by ethoxy, and $R_2$ and $R_3$ are as defined for formula I in claim 1.

**17.** A compound of formula IIa according to claim 16, wherein $R_1'$ is hydrogen, $C_1$-$C_6$alkyl or $C_3$-$C_5$cycloalkyl, or is phenyl that is mono- or di-substituted by halogen, $C_1$-$C_3$alkyl, methoxy or by ethoxy; and each of $R_2$ and $R_3$ is hydrogen or $C_1$-$C_4$alkyl.

**18.** A compound of formula IIa according to claim 17, wherein $R_1'$ is hydrogen, $C_1$-$C_4$alkyl or cyclopropyl; $R_2$ is hydrogen, methyl or ethyl; and $R_3$ is hydrogen or methyl.

**19.** A compound according to claim 18 of formula

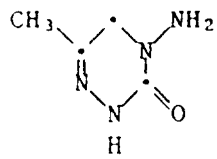

,

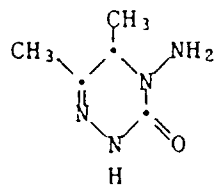

,

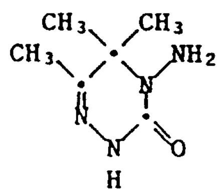

,

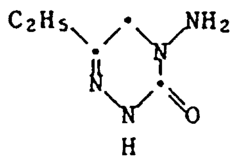

,

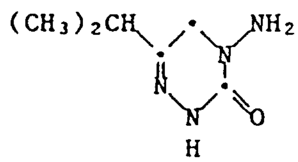

,

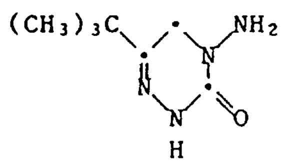

or

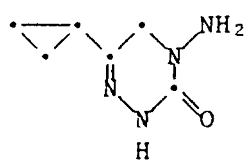

.

**20.** A compound of formula IV

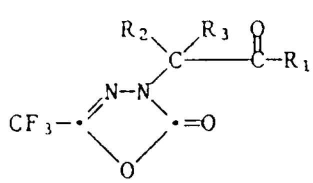

(IV)

wherein $R_1$, $R_2$ and $R_3$ are as defined for formula I in claim 1.

**21.** A compound of formula IV according to claim 20, wherein $R_1$ is hydrogen, $C_1$-$C_6$alkyl, $C_3$-$C_5$cycloalkyl, phenyl or phenyl that is mono- or di-substituted by halogen, $C_1$-$C_3$alkyl, methoxy or by ethoxy; and each of $R_2$ and $R_3$ is hydrogen or $C_1$-$C_4$alkyl.

**22.** A compound of formula IV according to claim 21, wherein $R_1$ is hydrogen, $C_1$-$C_4$alkyl or cyclopropyl; $R_2$ is hydrogen, methyl or ethyl; and $R_3$ is hydrogen or methyl.

**23.** A compound according to claim 22 of formula

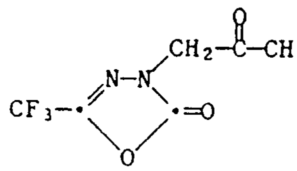

,

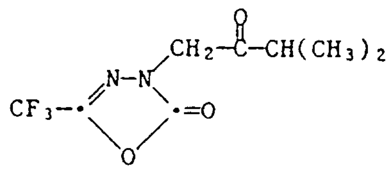

,

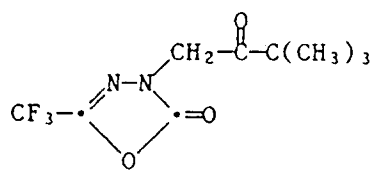

,

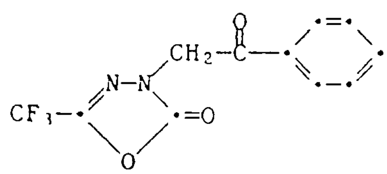

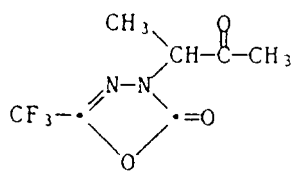

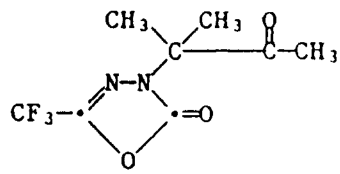

,

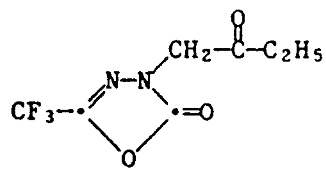

or

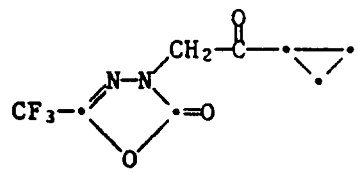

.

**Claims for the following Contracting State : ES**

**1.** A process for the preparation of a compound of formula

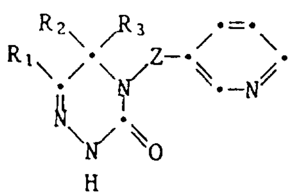

(I)

.

wherein
$R_1$ is hydrogen, $C_1$-$C_{12}$alkyl, $C_3$-$C_6$cycloalkyl, $C_1$-$C_4$-alkoxy-$C_1$-$C_6$alkyl, $C_1$-$C_2$haloalkyl, phenyl, benzyl, phenethyl, phenpropyl, phenbutyl or phenpentyl, or a phenyl, benzyl, phenethyl, phenpropyl, phenbutyl or phenpentyl radical that is mono- or di-substituted by halogen, $C_1$-$C_5$alkyl, $C_1$-$C_2$haloalkyl, methoxy and/or by ethoxy,
$R_2$ is hydrogen, $C_1$-$C_6$alkyl or $C_3$-$C_6$cycloalkyl, or is phenyl that is unsubstituted or substituted by $C_1$-$C_{12}$-alkyl, halogen or by $C_1$-$C_{12}$haloalkyl, or $R_1$ and $R_2$ together form a saturated or unsaturated 3- to 7-membered carbocycle,
$R_3$ is hydrogen or $C_1$-$C_6$alkyl, and
Z is -N=CH- or -NH-$CH_2$-,
or of a salt thereof with an organic or inorganic acid, which process comprises

A. reacting an aminotriazinone of formula

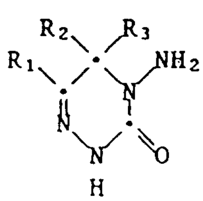

(II),

wherein $R_1$, $R_2$ and $R_3$ are as defined in formula I, with an aldehyde of formula

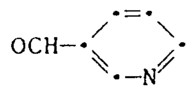

(III)

and, if desired,

B. converting the resulting pyridyl-methyleneaminotriazinone by selective reduction into pyridyl-methylamino-triazinone.

2. A process according to claim 1 for the preparation of a compound of formula I wherein $R_1$ is hydrogen, $C_1$-$C_6$-alkyl, $C_3$-$C_5$cycloalkyl, phenyl or phenyl that is mono- or di-substituted by halogen, $C_1$-$C_3$alkyl, methoxy or by ethoxy; each of $R_2$ and $R_3$ is hydrogen or $C_1$-$C_4$alkyl; and Z is -N=CH- or -NH-$CH_2$-.

3. A process according to claim 2 for the preparation of a compound of formula I wherein $R_1$ is hydrogen, $C_1$-$C_4$-alkyl, cyclopropyl or phenyl; $R_2$ is hydrogen, methyl or ethyl; $R_3$ is hydrogen or methyl; and Z is -N=CH-.

4. A process according to claim 2 for the preparation of a compound of formula I wherein $R_1$ is hydrogen, $C_1$-$C_4$-alkyl, cyclopropyl or phenyl; $R_2$ is hydrogen, methyl or ethyl; $R_3$ is hydrogen or methyl; and Z is -NH-$CH_2$-.

5. A process according to claim 3 for the preparation of a compound of formula

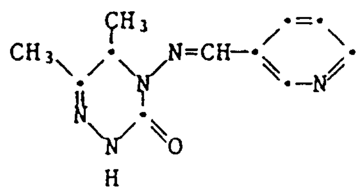

,

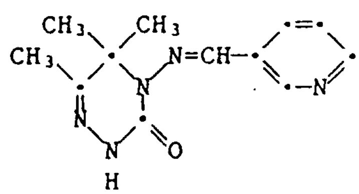

,

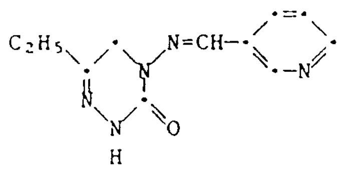

,

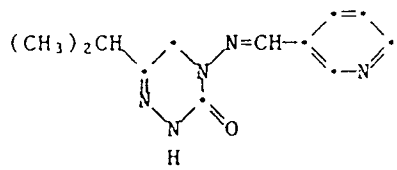

,

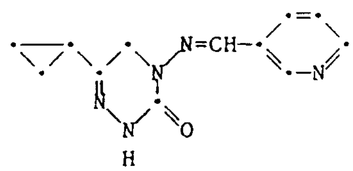

,

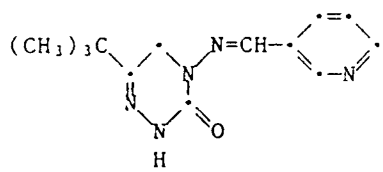

,

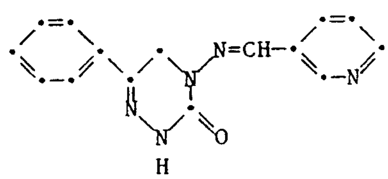

,

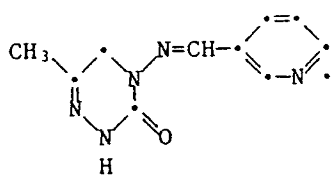 · HCl

,

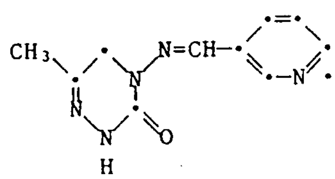 · 1/2 $H_2SO_4$

,

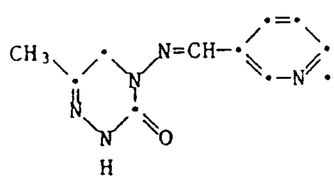 · $HNO_3$

,

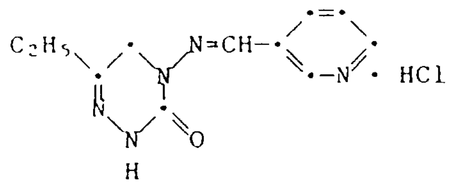

,

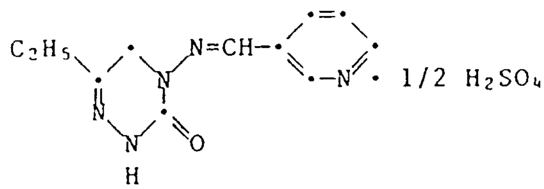

or

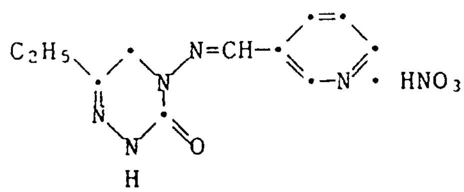

.

6. A process according to claim 3 for the preparation of a compound of formula

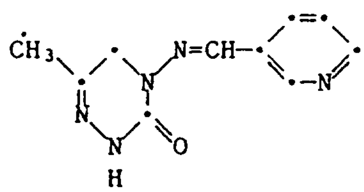

.

7. A process according to claim 4 for the preparation of a compound of formula

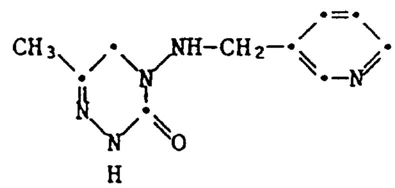

,

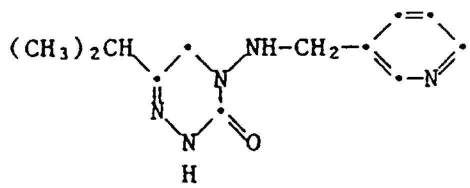

,

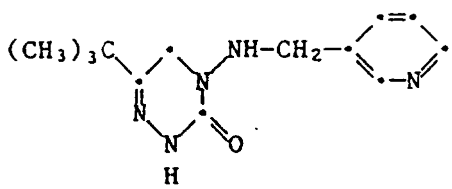

,

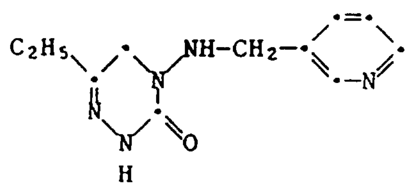

or

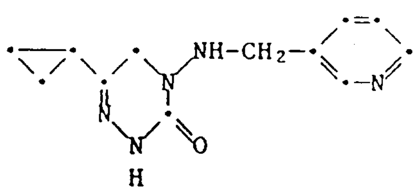

.

8. A pesticidal composition which comprises as active component a compound of formula I obtainable according to claim 1, or a salt thereof with an organic or inorganic acid, together with suitable carriers and/or adjuvants.

9. A pesticidal composition according to claim 8, which comprises as active component a compound obtainable according to any one of claims 2 to 7.

10. The use of a compound of formula I obtainable according to claim 1, or of a salt thereof with an organic or inorganic acid, for controlling pests on plants.

11. The use according to claim 10 for controlling insects and representatives of the order Acarina.

12. The use according to claim 11 for controlling plant-destructive insects.

13. The use according to claim 12 for controlling sucking insects.

14. A method of controlling pests on plants, which comprises bringing the pests in their various stages of development into contact with a compound of formula I obtainable according to claim 1, or with a salt thereof with an organic or inorganic acid.

**15.** A process for the preparation of a compound of formula

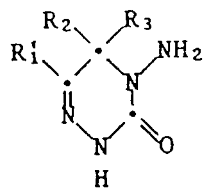

(IIa)

wherein
$R_1'$ is hydrogen, $C_1$-$C_{12}$alkyl, $C_3$-$C_6$cycloalkyl, $C_1$-$C_4$-alkoxy-$C_1$-$C_6$alkyl, $C_1$-$C_2$haloalkyl, benzyl, phenethyl, phenpropyl, phenbutyl or phenpentyl, or a phenyl, benzyl, phenethyl, phenpropyl, phenbutyl or phenpentyl radical that is mono- or di-substituted by halogen, $C_1$-$C_5$alkyl,$C_1$-$C_2$haloalkyl, methoxy and/or by ethoxy, and $R_2$ and $R_3$ are as defined for formula I in claim 1, which process comprises reacting a compound of formula

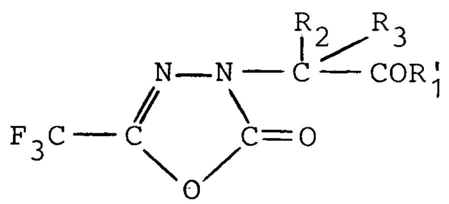

(IV),

wherein $R_1'$ , $R_2$ and $R_3$ are as defined for formula IIa, with hydrazine hydrate.

**16.** A process according to claim 15 for the preparation of a compound of formula IIa wherein $R_1'$ is hydrogen, $C_1$-$C_6$alkyl or $C_3$-$C_5$cycloalkyl, or is phenyl that is mono-or di-substituted by halogen, $C_1$-$C_3$alkyl, methoxy or by ethoxy; and each of $R_2$ and $R_3$ is hydrogen or $C_1$-$C_4$alkyl.

**17.** A process according to claim 16 for the preparation of a compound of formula IIa wherein $R_1'$ is hydrogen, $C_1$-$C_4$alkyl or cyclopropyl; $R_2$ is hydrogen, methyl or ethyl; and $R_3$ is hydrogen or methyl.

**18.** A process according to claim 17 for the preparation of a compound of formula

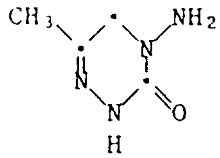

,

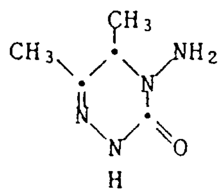

,

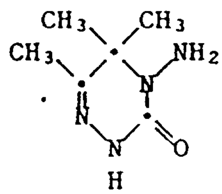

,

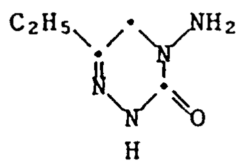

,

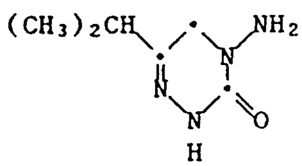

,

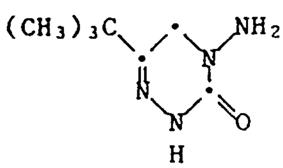

or

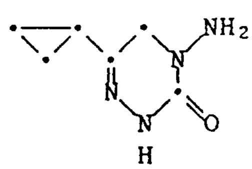

.

**19.** A process for the preparation of a compound of formula

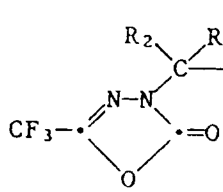

(IVa)

wherein $R_1$, $R_2$ and $R_3$ are as defined for formula I in claim 1, which process comprises reacting a

compound of formula

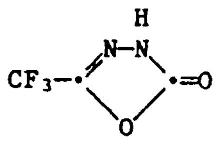

(V)

with a compound of formula

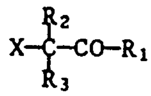

(VI),

wherein $R_1$, $R_2$ and $R_3$ are as defined for formula I, claim 1, and X is halogen.

**20.** A process according to claim 19 for the preparation of a compound of formula IVa wherein $R_1$ is hydrogen, $C_1$-$C_6$alkyl, $C_3$-$C_5$cycloalkyl, phenyl or phenyl that is mono- or di-substituted by halogen, $C_1$-$C_3$alkyl, methoxy or by ethoxy; and each of $R_2$ and $R_3$ is hydrogen or $C_1$-$C_4$alkyl.

**21.** A process according to claim 20 for the preparation of a compound of formula IVa wherein $R_1$ is hydrogen, $C_1$-$C_4$alkyl or cyclopropyl; $R_2$ is hydrogen, methyl or ethyl; and $R_3$ is hydrogen or methyl.

**22.** A process according to claim 21 for the preparation of a compound of formula

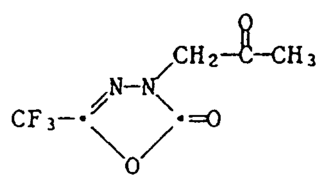

,

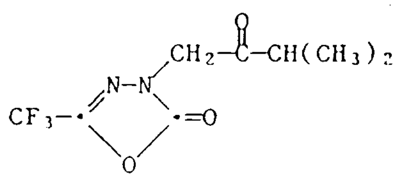

,

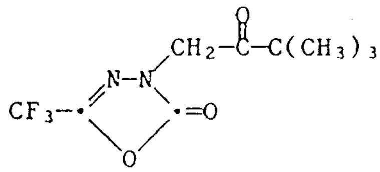

,

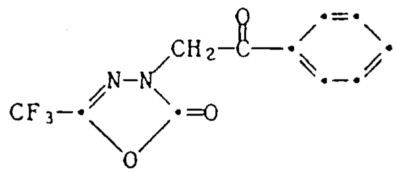

,

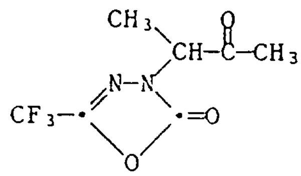

,

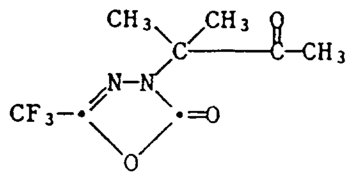

,

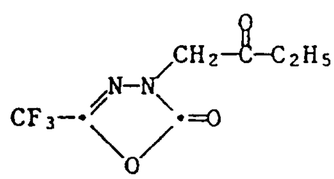

or

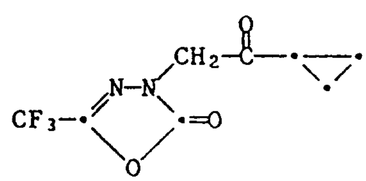

.

**Revendications**

**Revendications pour les Etats contractants suivants : AT, BE, CH, DE, FR, GB, IT, LI, NL**

**1.** Composés de formule I

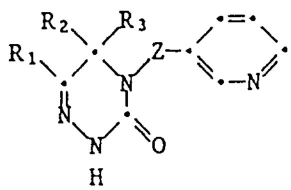

(I)

où

- $R_1$ représente l'hydrogène, un alkyle en $C_1$-$C_{12}$, un cycloalkyle en $C_3$-$C_6$, un alcoxy en $C_1$-$C_4$ alkyle en $C_1$-$C_6$, un halogénoalkyle en $C_1$-$C_2$, le phényle, le benzyle, le phénéthyle, le phénpropyle, le phénbutyle, le phénpentyle ou un reste phényle, benzyle, phénéthyle, phénpropyle, phénbutyle ou phénpentyle mono- ou disubstitué par un halogène, un alkyle en $C_1$-$C_5$, un halogénoalkyle en $C_1$-$C_2$, le méthoxy et/ou l'éthoxy.
- $R_2$ représente l'hydrogène, un alkyle en $C_1$-$C_6$, un cycloalkyle en $C_3$-$C_6$, un phényle non substitué ou substitué par un alkyle en $C_1$-$C_{12}$, un halogène ou un halogènoalkyle en $C_1$-$C_{12}$ ou $R_1$ et $R_2$ forment ensemble un carbocycle ayant 3 à 7 chaînons saturé ou insaturé,
- $R_3$ représente l'hydrogène ou un alkyle en $C_1$-$C_6$ et
- Z représente -N=CH- ou -NH-$CH_2$-

ainsi que leurs sels avec des acides organiques ou minéraux.

**2.** Composés de formule I selon la revendication 1 où $R_1$ représente l'hydrogène, un alkyle en $C_1$-$C_6$, un cycloalkyle en $C_3$-$C_5$, le phényle ou un phényle mono- ou disubstitué par un halogène, un alkyle en $C_1$-$C_3$, le méthoxy ou l'éthoxy ; $R_2$ et $R_3$ représentent l'hydrogène ou un alkyle en $C_1$-$C_4$ et Z représente -N=CH- ou -NH-$CH_2$-.

**3.** Composés de formule I selon la revendication 2 où $R_1$ représente l'hydrogène, un alkyle en $C_1$-$C_4$, le cyclopropyle ou le phényle ; $R_2$ représente l'hydrogène, le méthyle ou l'éthyle ; $R_3$ représente l'hydrogène ou le méthyle ; et Z représente -N=CH-.

**4.** Composés de formule I selon la revendication 2 où $R_1$ représente l'hydrogène, un alkyle en $C_1$-$C_4$, le cyclopropyle ou le phényle ; $R_2$ représente l'hydrogène, le méthyle ou l'éthyle ; $R_3$ représente l'hydrogène ou le méthyle ; et Z représente -NH-$CH_2$-.

**5.** Un composé selon la revendication 3 de formule

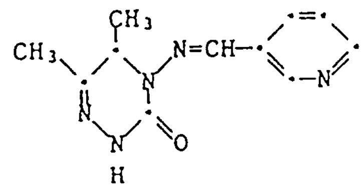

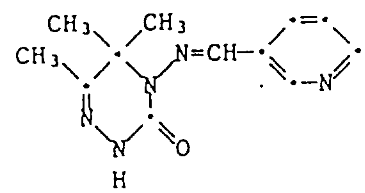

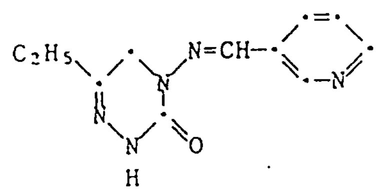

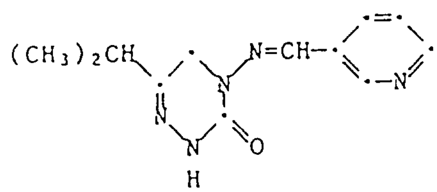

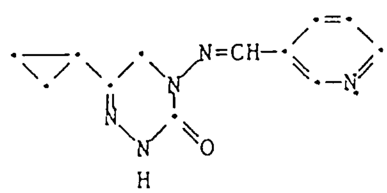

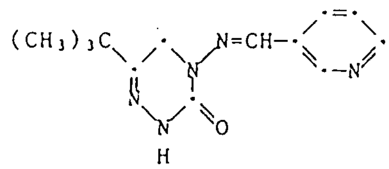

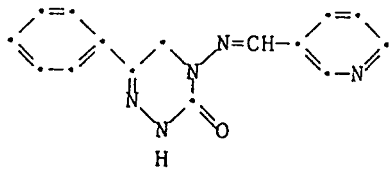

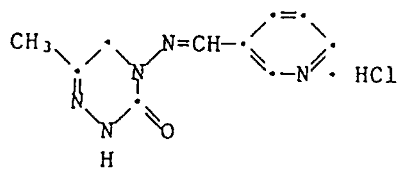

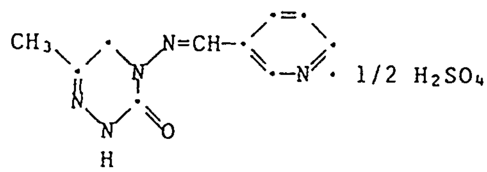

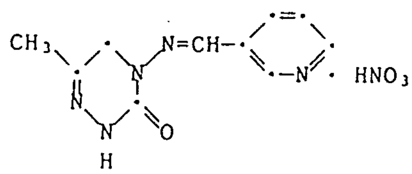

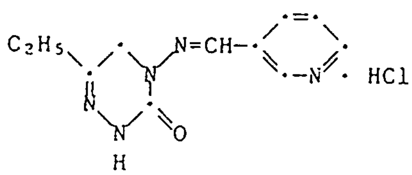

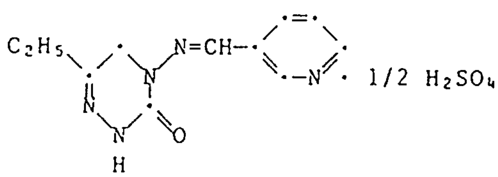

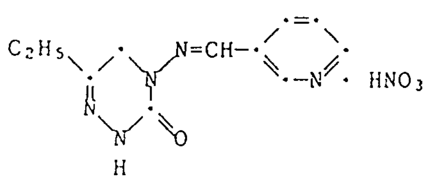

**6.** Le composé selon la revendication 3 de formule

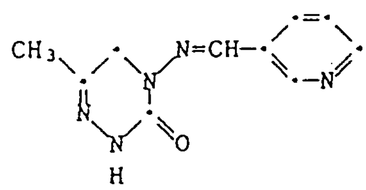

**7.** Un composé selon la revendication 4 de formule

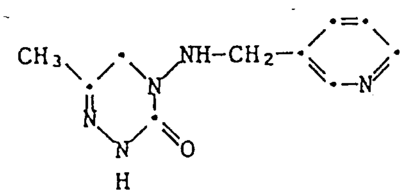

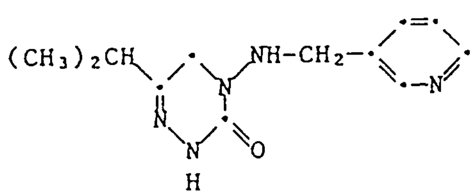

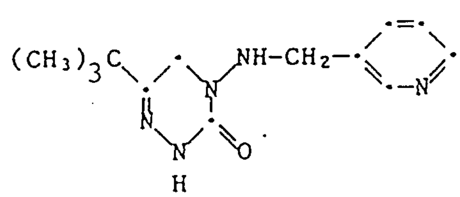

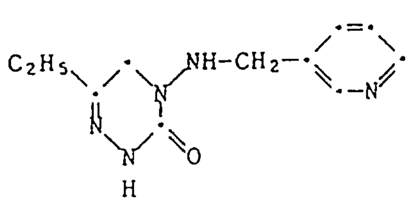

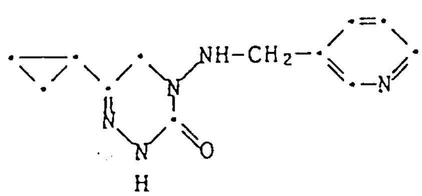

**8.** Procédé pour la préparation d'un composé de formule

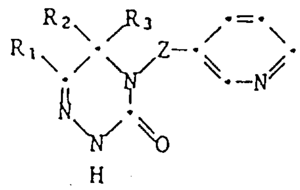

(I)

où $Z_1$, $R_1$, $R_2$ et $R_3$ ont les significations indiquées dans la revendication 1, caractérisé

A. en ce que l'on fait réagir une aminotriazinone de formule II

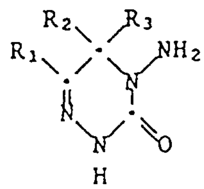

(II)

où $R_1$, $R_2$ et $R_3$ ont les significations indiquées pour la formule I, avec l'aldéhyde de formule III

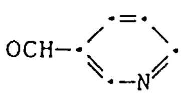

(III)

et éventuellement

B. en ce que l'on transforme la pyridyl-méthylèneaminotriazinone obtenue par réduction sélective en la pyridylméthylamino-triazinone.

**9.** Composition pour lutter contre les parasites contenant, comme composant actif, un composé de formule I selon la revendication 1 ou l'un de ses sels avec un acide organique ou minéral, associé à des supports et/ou à des additifs appropriés.

**10.** Composition pour lutter contre les parasites selon la revendication 9 contenant, comme composant actif, un composé selon l'une des revendications 2 à 7.

**11.** Utilisation d'un composé de formule I selon la revendication 1 ou de l'un de ses sels avec un acide organique ou minéral pour lutter contre les parasites sur les plantes.

**12.** Utilisation selon la revendication 11 pour lutter contre les insectes et les représentants de l'ordre des acariens.

**13.** Utilisation selon la revendication 12 pour lutter contre les insectes causant des dommages aux plantes.

**14.** Utilisation selon la revendication 13 pour lutter contre les insectes suceurs.

**15.** Procédé pour lutter contre les parasites des plantes, caractérisé en ce que l'on met en contact les parasites dans leurs différents stades de développement avec un composé de formule I selon la revendication 1, ou avec l'un de ses sels qu'il forme avec un acide organique ou minéral.

**16.** Composés de formule IIa

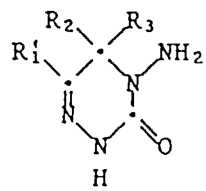

(IIa)

où
R'$_1$ représente l'hydrogène, un alkyle en $C_1$-$C_{12}$, un cycloalkyle en $C_3$-$C_6$, un alcoxy en $C_1$-$C_4$ alkyle en $C_1$-$C_6$, un halogénoalkyle en $C_1$-$C_2$, un phényle, le benzyle, le phénéthyle, le phénpropyle, le phénbutyle, le phénpentyle ou le reste phényle, benzyle, phénéthyle, phénpropyle, phénbutyle ou phénpentyle mono- ou disubstitué par un halogène, un alkyle en $C_1$-$C_5$, un halogénoalkyle en $C_1$-$C_2$, le méthoxy et/ou l'éthoxy,
et $R_2$ et $R_3$ ont les significations indiquées pour la formule I dans la revendication 1.

**17.** Composés de formule IIa selon la revendication 16, où R'$_1$ représente l'hydrogène, un alkyle en $C_1$-$C_4$, un cycloalkyle en $C_3$-$C_5$ ou un phényle mono- ou disubstitué par un halogéne, un alkyle en $C_1$-$C_3$, le méthoxy ou l'éthoxy ; et $R_2$ et $R_3$ représentent l'hydrogène ou un alkyle en $C_1$-$C_4$.

**18.** Composés de formule IIa selon la revendication 17 où R'$_1$ représente l'hydrogène, un alkyle en $C_1$-$C_4$ ou le cyclopropyle ; $R_2$ représente l'hydrogène, le méthyle ou l'éthyle, et $R_3$ représente l'hydrogène ou le méthyle.

**19.** Les composés selon la revendication 18 de formules

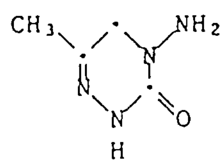

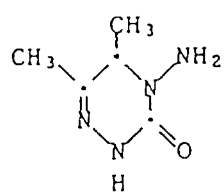

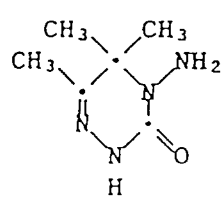

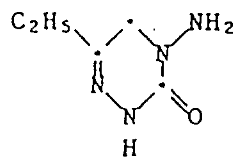

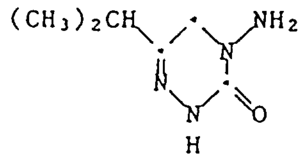

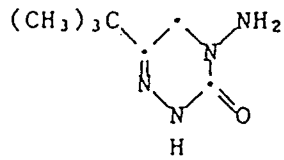

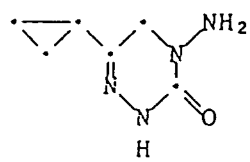

**20.** Composés de formule IV

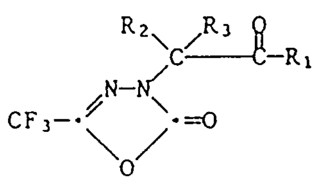

(IV)

où $R_1$, $R_2$ et $R_3$ ont les significations indiquées pour la formule (I) dans la revendication 1.

**21.** Composés de formule IV selon la revendication 20, où $R_1$ représente l'hydrogène, un alkyle en $C_1$-$C_6$, un cycloalkyle en $C_3$-$C_5$, le phényle ou un phényle mono- ou disubstitué par un halogène, un alkyle en $C_1$-$C_3$, le méthoxy ou l'éthoxy ; et $R_2$ et $R_3$ représentent l'hydrogène ou un alkyle en $C_1$-$C_4$.

**22.** Composés de formule IV selon la revendication 21, où $R_1$ représente l'hydrogène, un alkyle en $C_1$-$C_4$ ou le cyclopropyle ; $R_2$ représente l'hydrogène, le méthyle ou l'éthyle ; et $R_3$ représente l'hydrogène ou le méthyle.

**23.** les composés selon la revendication 22 de formules

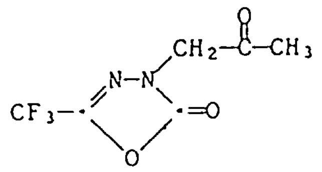

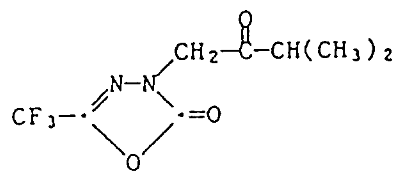

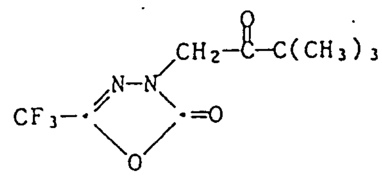

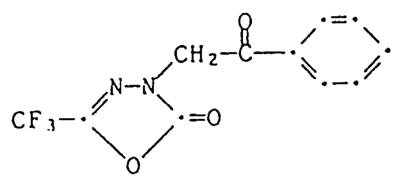

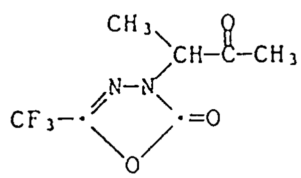

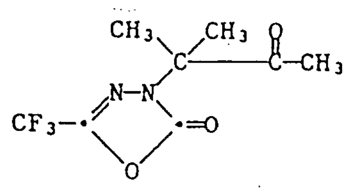

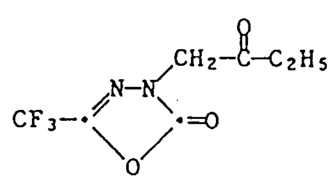

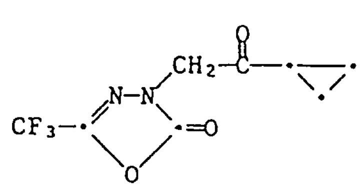

**Revendications pour l'Etat contractant suivant : ES**

1. Procédé pour la préparation d'un composé de formule

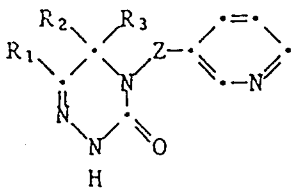

(I)

où

- $R_1$ représente l'hydrogène, un alkyle en $C_1$-$C_{12}$, un cycloalkyle en $C_3$-$C_6$, un alcoxy en $C_1$-$C_4$ alkyle en $C_1$-$C_6$, un halogénoalkyle en $C_1$-$C_2$, le phényle, le benzyle, le phénéthyle, le phénpropyle, le phénbutyle, le phénpentyle ou un reste phényle, benzyle, phénéthyle, phénpropyle, phénbutyle ou phénpentyle mono- ou disubstitué par un halogène, un alkyle en $C_1$-$C_5$, un halogénoalkyle en $C_1$-$C_2$, le méthoxy et/ou l'éthoxy,
- $R_2$ représente l'hydrogène, un alkyle en $C_1$-$C_6$, un cycloalkyle en $C_3$-$C_6$, un phényle non substitué ou substitué par un alkyle en $C_1$-$C_{12}$, un halogène ou un halogénoalkyle en $C_1$-$C_{12}$ ou $R_1$ et $R_2$ forment ensemble un carbocycle ayant 3 à 7 chaînons saturé ou insaturé,
- $R_3$ représente l'hydrogène ou un alkyle en $C_1$-$C_6$ et
- Z représente -N=CH- ou -NH-$CH_2$-

ainsi que leurs sels avec des acides organiques ou minéraux,
caractérisé

A. en ce que l'on fait réagir une aminotriazinone de formule

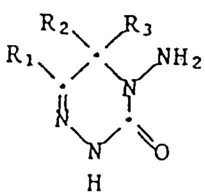

(II)

où $R_1$, $R_2$ et $R_3$ ont les significations indiquées pour la formule I, avec l'aldéhyde de formule

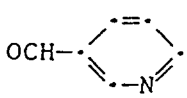

(III)

et éventuellement

B. en ce que l'on transforme la pyridyl-méthylèneaminotriazinone obtenue par réduction sélective en la pyridyl-méthylamino-triazinone.

2. Procédé selon la revendication 1 pour la préparation d'un composé de formule I où $R_1$ représente l'hydrogène, un alkyle en $C_1$-$C_6$, un cycloalkyle en $C_3$-$C_5$, le phényle ou un phényle mono- ou disubstitué par un halogène, un alkyle en $C_1$-$C_3$, le méthoxy ou l'éthoxy ; $R_2$ et $R_3$ représentent l'hydrogène ou un alkyle en $C_1$-$C_4$ et Z représente -N=CH-ou -NH-$CH_2$-.

3. Procédé selon la revendication 2 pour la préparation d'un composé de formule I où $R_1$ représente l'hydrogène, un alkyle en $C_1$-$C_4$, le cyclopropyle ou le phényle ; $R_2$ représente l'hydrogène, le méthyle ou l'éthyle ; $R_3$ représente l'hydrogène ou le méthyle ; et Z représente -N=CH-.

4. Procédé selon la revendication 2 pour la préparation d'un composé de formule I où $R_1$ représente l'hydrogène, un alkyle en $C_1$-$C_4$, le cyclopropyle ou le phényle ; $R_2$ représente l'hydrogène, le méthyle ou l'éthyle ; $R_3$ représente l'hydrogène ou le méthyle; et Z représente -NH-$CH_2$-.

5. Procédé selon la revendication 3 pour la préparation d'un composé de formule

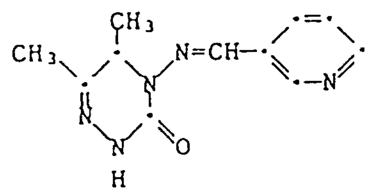

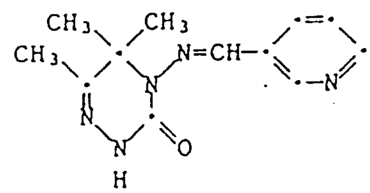

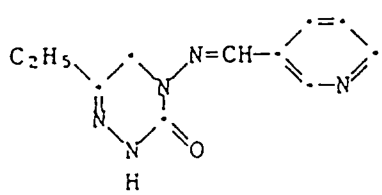

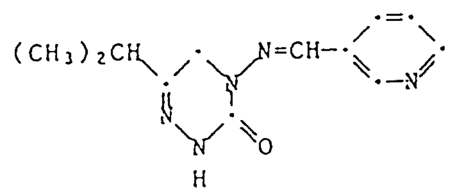

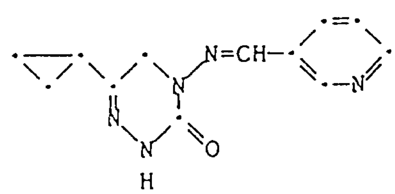

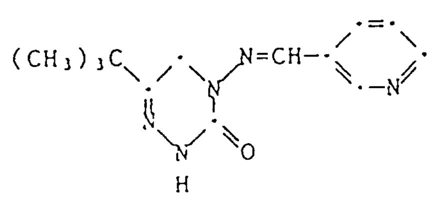

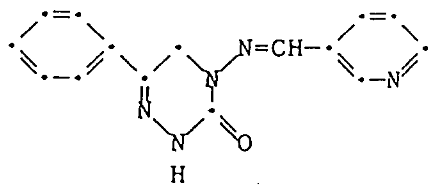

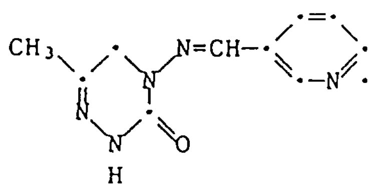
· HCl

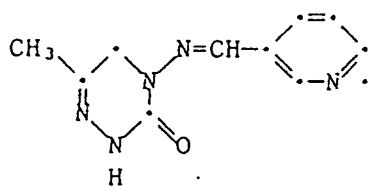
· 1/2 $H_2SO_4$

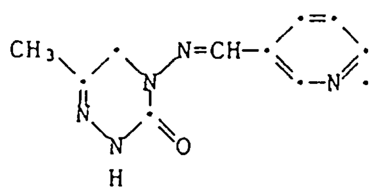
· $HNO_3$

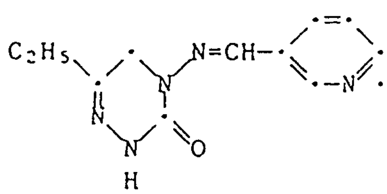
· HCl

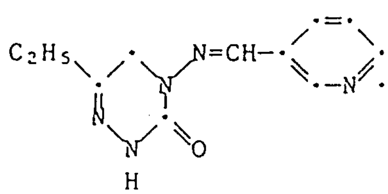
· 1/2 $H_2SO_4$

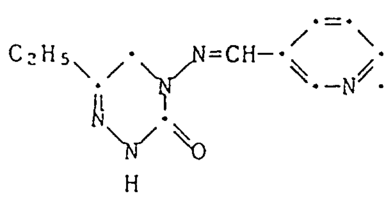
· $HNO_3$

6. Procédé selon la revendication 3 pour la préparation d'un composé de formule

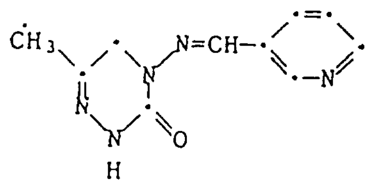

7. Procédé selon la revendication 4 pour la préparation d'un composé de formule

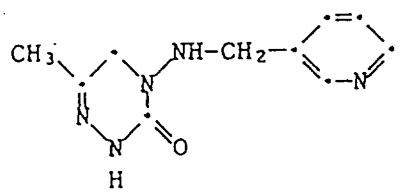

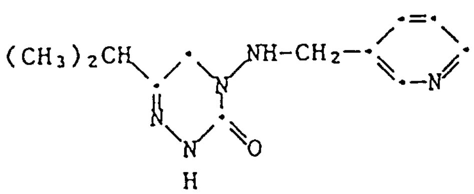

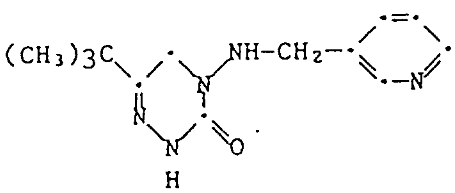

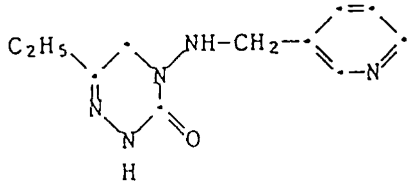

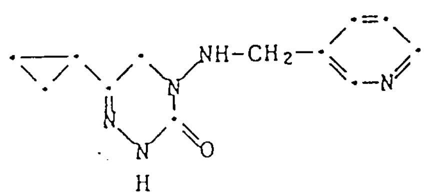

8. Composition pour lutter contre les parasites contenant, comme composant actif, un composé de formule I obtenu selon la revendication 1 ou l'un de ses sels avec un acide organique ou minéral, associé à des supports et/ou à des additifs appropriés.

**9.** Composition pour lutter contre les parasites selon la revendication 8 contenant, comme composant actif, un composé obtenu selon l'une des revendications 2 à 7.

**10.** Utilisation d'un composé de formule I obtenu selon la revendication 1, ou de l'un de ses sels avec un acide organique ou minéral pour lutter contre les parasites des plantes.

**11.** Utilisation selon la revendication 10 pour lutter contre les insectes et les représentants de l'ordre des acariens.

**12.** Utilisation selon la revendication 11 pour lutter contre les insectes causant des dommages aux plantes.

**13.** Utilisation selon la revendication 12 pour lutter contre les insectes suceurs.

**14.** Procédé pour lutter contre les parasites des plantes, caractérisé en ce que l'on met en contact les parasites dans leurs différents stades de développement avec un composé de formule I obtenu selon la revendication 1 ou avec l'un de ses sels qu'il forme avec un acide organique ou minéral.

**15.** Procédé pour la préparation d'un composé de formule

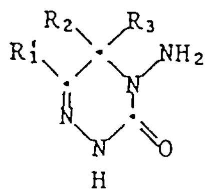

(IIa)

où
R'$_1$ représente l'hydrogène, un alkyle en $C_1$-$C_{12}$, un cycloalkyle en $C_3$-$C_6$, un alcoxy en $C_1$-$C_4$ alkyle en $C_1$-$C_6$, un halogénoalkyle en $C_1$-$C_2$, le benzyle, le phénéthyle, le phénpropyle, le phénbutyle, le phénpentyle ou le reste phényle, benzyle. phénéthyle, phénpropyle, phénbutyle ou phénpentyle mono- ou disubstitué par un halogène, un alkyle en $C_1$-$C_5$, un halogénoalkyle en $C_1$-$C_2$, le méthoxy et/ou l'éthoxy et
$R_2$ et $R_3$ ont les significations indiquées pour la formule I dans la revendication 1, caractérisé en ce que l'on fait réagir un composé de formule

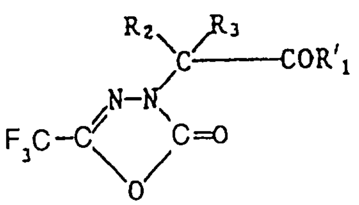

(IV)

où R'$_1$, $R_2$ et $R_3$ ont les significations indiquées pour la formule IIa, avec l'hydrate d'hydrazine.

**16.** Procédé selon la revendication 15 pour la préparation d'un composé de formule IIa où R'$_1$ représente l'hydrogène, un alkyle en $C_1$-$C_6$, un cycloalkyle en $C_3$-$C_5$ ou un phényle mono- ou disubstitué par un halogène, un alkyle en $C_1$-$C_3$, le méthoxy ou l'éthoxy ; et $R_2$ et $R_3$ représentent l'hydrogène ou un alkyle en $C_1$-$C_4$.

**17.** Procédé selon la revendication 16 pour la préparation d'un composé de formule IIa où R'$_1$ représente l'hydrogène, un alkyle en $C_1$-$C_4$ ou le cyclopropyle ; $R_2$ représente l'hydrogène, le méthyle ou l'éthyle ; et $R_3$ représente l'hydrogène ou le méthyle.

**18.** Procédé selon la revendication 17 pour la préparation d'un composé de formule

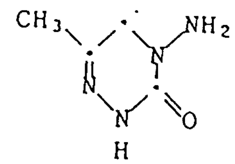

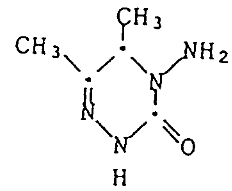

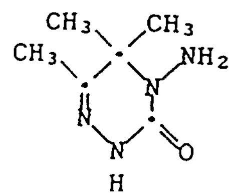

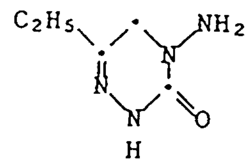

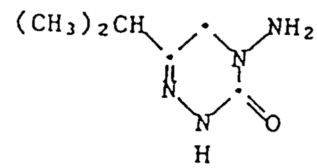

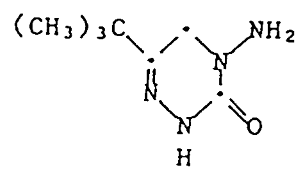

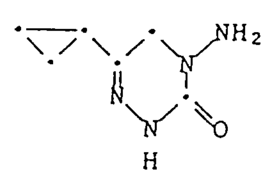

**19.** Procédé pour la préparation d'un composé de formule

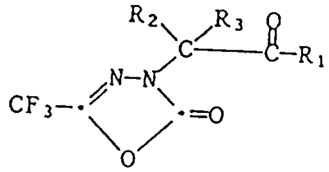

(IVa)

où $R_1$, $R_2$ et $R_3$ ont les significations indiquées pour la formule I dans la revendication 1, caractérisé en ce que l'on fait réagir un composé de formule

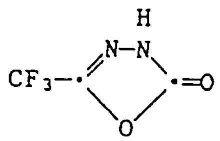

(V)

avec un composé de formule

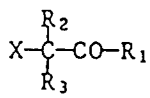

(VI)

où $R_1$, $R_2$ et $R_3$ ont les significations indiquées pour la formule I dans la revendication 1 et X représente un halogène.

**20.** Procédé selon la revendication 19 pour la préparation d'un composé de formule IVa où $R_1$ représente l'hydrogène, un alkyle en $C_1$-$C_6$, un cycloalkyle en $C_3$-$C_5$, le phényl ou un phényle mono- ou disubstitué par un halogène, un alkyle en $C_1$-$C_3$, le méthoxy ou l'éthoxy ; et $R_2$ et $R_3$ représentent l'hydrogène ou un alkyle en $C_1$-$C_4$.

**21.** Procédé selon la revendication 20 pour la préparation d'un composé de formule IVa où $R_1$ représente l'hydrogène, un alkyle en $C_1$-$C_4$ ou le cyclopropyle ; $X_2$ représente l'hydrogène, le méthyle ou l'éthyle ; et $R_3$ représente l'hydrogène ou le méthyle.

**22.** Procédé selon la revendication 21 pour la préparation d'un composé de formule